# EUROPEAN PATENT SPECIFICATION

(11) **EP 1 585 966 B1**
(45) Date of publication and mention of the grant of the patent: **30.11.2011**
(21) Application number: 03755724.6
(22) Date of filing: 11.07.2003
(51) Int. Cl.: A61K 39/395

(54) **Treatment of cancer with the anti-ErbB2 antibody rhuMAb 2C4**
Behandlung von Krebs mit dem anti-ErbB2-Antikörper rhuMAb 2C4
Traitment de cancer par l'anticorps rhuMAb 2C4

(30) Priority: 15.07.2002 US 396290 P; 20.06.2003 US 480043 P
(43) Date of publication of application: 19.10.2005
(60) Divisional application: 10176072.6
(73) Proprietor: Genentech, Inc., South San Francisco CA 94080-4990 (US); F.Hoffmann-La Roche AG, 4070 Basel (CH)
(72) Inventor: KOLL, Hans, 85247 Oberroth (DE); BOSSENMAIER, Birgit, 82229 Seefeld (DE); MÜLLER, Hans-Joachim, 82377 Penzberg (DE); SLIWKOWSKI, Mark, X., San Carlos, CA 94070 (US); KELSEY, Stephen, Michael, Montara, CA 94037 (US)
(74) Representative: Walton, Seán Malcolm
(86) International application number: PCT/US2003/021590
(87) International publication number: WO 2004/008099

(56) References cited:
- WO-A-01/00245
- WO-A-01/09186
- WO-A-01/89566
- US-A- 5 811 098
- US-A- 5 811 098
- US-A1- 2002 064 805
- US-B1- 6 417 168
- US-B1- 6 417 168
- XIA W ET AL: "Combination of EGFR, HER-2/neu, and HER-3 is a stronger predictor for the outcome of oral squamous cell carcinoma than any individual family members." CLINICAL CANCER RESEARCH : AN OFFICIAL JOURNAL OF THE AMERICAN ASSOCIATION FOR CANCER RESEARCH. DEC 1999, vol. 5, no. 12, December 1999 (1999-12), pages 4164-4174, XP002400085 ISSN: 1078-0432
- GILBERTSON RICHARD J ET AL: "Prognostic significance of HER2 and HER4 coexpression in childhood medulloblastoma" CANCER RESEARCH, vol. 57, no. 15, 1997, pages 3272-3280, XP008069241 ISSN: 0008-5472
- LANE HEIDI A ET AL: "ErbB2 potentiates breast tumor proliferation through modulation of p27Kip1-Cdk2 complex formation: Receptor overexpression does not determine growth dependency" MOLECULAR AND CELLULAR BIOLOGY, vol. 20, no. 9, May 2000 (2000-05), pages 3210-3223, XP002400087 ISSN: 0270-7306
- LYNE J C ET AL: "Tissue expression of neu differentiation factor/heregulin and its receptor complex in prostate cancer and its biologic effects on prostate cancer cells in vitro." THE CANCER JOURNAL FROM SCIENTIFIC AMERICAN. 1997 JAN-FEB, vol. 3, no. 1, January 1997 (1997-01), pages 21-30, XP002400088 ISSN: 1081-4442
- OLAYIOYE M: "The Erbbeta Signaling Network: Receptor Heterodimerization in Development and Cancer" EMBO JOURNAL, OXFORD UNIVERSITY PRESS, SURREY, GB, vol. 19, no. 13, January 2000 (2000-01), pages 3159-3167, XP002985171 ISSN: 0261-4189
- MOTOYAMA ANDREA B ET AL: "The efficacy of ErbB receptor-targeted anticancer therapeutics is influenced by the availability of epidermal growth factor-related peptides" CANCER RESEARCH, vol. 62, no. 11, 11 June 2002 (2002-06-11), pages 3151-3158, XP001155498 ISSN: 0008-5472
- CHEN XINMEI ET AL: "Enhanced drug resistance in cells coexpressing ErbB2 with EGF receptor or ErbB3" BIOCHEMICAL AND BIOPHYSICAL RESEARCH COMMUNICATIONS, vol. 277, no. 3, 2 November 2000 (2000-11-02), pages 757-763, XP002400090 ISSN: 0006-291X
- BROCKHOFF GERO ET AL: "Epidermal growth factor receptor, c-erbB2 and c-erbB3 receptor interaction, and related cell cycle kinetics of SK-BR-3 and BT474 breast carcinoma cells" CYTOMETRY, vol. 44, no. 4, 1 August 2001 (2001-08-01), pages 338-348, XP002400091 ISSN: 0196-4763
- NEVE RICHARD M ET AL: "Effects of oncogenic ErbB2 on G1 cell cycle regulators in breast tumour cells" ONCOGENE, vol. 19, no. 13, 23 March 2000 (2000-03-23), pages 1647-1656, XP002400092 ISSN: 0950-9232
- AGUS D.B. ET AL: 'Targeting ligand-activated ErbB2 signaling inhibits breast and prostate tumor growth' CANCER CELL vol. 2, August 2002, pages 127 - 137, XP002988666
- TZAHAR E. ET AL: 'The ErbB-2/HER2 oncogenic receptor of adenocarcinomas: from orphanhood to multiple stromal ligands' BIOCHEMICA ET BIOPHYSICA ACTA vol. 1377, 20 February 1998, pages M29 - M34, XP004281795
- SLIKOWSKI M.X. ET AL: 'Coexpression of erbB2 and erbB3 proteins reconstitutes a high affinity receptor for heregulin' THE JOURNAL OF BIOLOGICAL CHEMISTRY vol. 269, no. 20, 20 May 1994, pages 14661 - 14665, XP000605388
- QIAN X. ET AL: 'p185c-neu and Epidermal Growth Factor Receptor Associated into Structure Composed of Activated Kinases' PROCEEDINGS OF THE NATIONAL ACADEMY OF SCIENCES vol. 89, February 1992, pages 1330 - 1334, XP002988667
- GORDON MICHAEL S ET AL: "Clinical activity of pertuzumab (rhuMAb 2C4), a HER dimerization inhibitor, in advanced ovarian cancer: potential predictive relationship with tumor HER2 activation status", JOURNAL OF CLINICAL ONCOLOGY, AMERICAN SOCIETY OF CLINICAL ONCOLOGY, US, vol. 24, no. 26, 10 September 2006 (2006-09-10), pages 4324-4332, XP002492090, ISSN: 0732-183X, DOI: DOI:10.1200/JCO.2005.05.4221 [retrieved on 2006-08-07]
- BASELGA, J. ET AL.: "Objective response rate in a phase II multicenter trial of pertuzumab (P), a HER2 dimerization inhibiting monoclonal antibody, in combination with trastuuzumab (T) in patients (pts) with HER2-positive metastatic breast cancer (MBC) which as progressed during treatment with T", JOURNAL OF CLINICAL ONCOLOGY, vol. 25, no. 18S, 20 June 2007 (2007-06-20), page 1004,
- MAKHIJA, S. ET AL.: "Results from a phase II randomzied, placebo-controlled, double-blind trial suggest improved PFS with the addition of pertuzumab to gemcitabine in patients with platinum-resistant ovarian, fallopian tube, or primary peritoneal cancer", JOURNAL OF CLINICAL ONCOLOGY, vol. 25, no. 18S, 20 June 2007 (2007-06-20), page 5507,
- CHEE M NG ET AL: "Rationale for Fixed Dosing of Pertuzumab in Cancer Patients Based on Population Pharmacokinetic Analysis", PHARMACEUTICAL RESEARCH, KLUWER ACADEMIC PUBLISHERS-PLENUM PUBLISHERS, NL, vol. 23, no. 6, 26 May 2006 (2006-05-26), pages 1275-1284, XP019405128, ISSN: 1573-904X, DOI: DOI:10.1007/S11095-006-0205-X
- ALLISON, D. ET AL,: "Pharmacokinetics (PK) of Pertuzumab (rhuMAb 2C4) in Phase II Studies of Ovarian, Breast, Prostate, and Lung Cancers", , 2005, page 2532,

## Description

### Field of the Invention

The present invention relates to the recombinant humanized anti-HER2 antibody 2C4 (rhuMab 2C4) for use in a method of treating patients suffering from tumors.

### Background of the Invention

The ErbB family of receptor tyrosine kinases are important mediators of cell growth, differentiation and survivaL The receptor family includes four distinct members including epidermal growth factor receptor (EGFR or ErbB1), HER2 (ErbB2 or p185*^{neu}*), HBR3 (ErbB3) and HER4 (ErbB4 or tyro2).

EGFR, encoded by the erbB1 gene, has been causally implicated in human malignancy. In particular, increased expression of EGFR has been observed in breast, bladder, lung, head, neck and stomach cancer as well as glioblastomas. Increased EGFR receptor expression is often associated with increased production of the EGFR ligand, transforming growth factor alpha (TGF-α), by the same tumor cells resulting in receptor activation by an autocrine stimulatory pathway. Baselga and Mendelsohn Pharmac. *Ther*., 64:127-154 (1994). In addition, an epidermal growth factor receptor related protein (ERRP) wherein a cDNA fragment clone of 1583 base pairs with 90-95% sequence homology to mouse EGFR and a truncated rat EGFR has been described (US Patent No. 6,399,743; and US Publication No. 2003/0096373). Monoclonal antibodies directed against the EGFR or its ligands, TGF-α and EGF, have been evaluated as therapeutic agents in the treatment of such malignancies. See, e.g., Baselga and Mendelsohn., supra; Masui et al., Cancer Research, 44:1002-1007 (1984); and Wu et al., J. Clin. Invest., 95:1897-1905 (1995).

The second member of the ErbB family, p185neu, was originally identified as the product of the transforming gene from neuroblastomas of chemically treated rats. The activated form of the neu proto-oncogene results from a point mutation (valine to glutamic acid) in the transmembrane region of the encoded protein. Amplification of the human homolog of neu (HBR2) is observed in breast and ovarian cancers and correlates with a poor prognosis (Slamon et al., Science, 235:177-182 (1987); Slamon et aL, Science, 244:707-712 (1989); and US Patent No. 4,968,603). To date, no point mutation analogous to that in the neu proto-oncogene has been reported for human tumors. Overexpression of ErbB2 (frequently but not uniformly due to gene amplification) has also been observed in other carcinomas including carcinomas of the stomach, endometrium, salivary gland, lung, kidney, colon, thyroid, pancreas and bladder. See, among others, King et al., Science, 229:974 (1985); Yokota et al., Lancet, 1:765-767 (1986); Fukushigi et al., Mol Cell Biol., 6:955-958 (1986); Geurin et al., Oncogene Res., 3:21-31 (1988); Cohen et al., Oncogene, 4:81-88 (1989); Yonemura et al., Cancer Res., 51:1034 (1991); Borst et al., Gynecol. Oncol., 38:364 (1990); Weiner et al., Cancer Re., 50:421-425 (1990); Kern et al., Cancer Res., 50:5184 (1990); Park et al., Cancer Res., 49:6605 (1989); Zhau et al., Mol. Carcinog., 3:354-357 (1990); Aasland et al., Br. J. Cancer, 57:358-363 (1988); Williams et al., Pathiobiology, 59:46-52 (1991); and McCann et al., Cancer, 65:88-92 (1990). ErbB2 may be overexpressed in prostate cancer (Gu et al., Cancer Lett., 99:185-9 (1996); Ross et al., Hum. Pathol., 28:827-33 (1997); Ross et al., Cancer, 79:2162-70 (1997); and Sadasivan et al., J. Urol., 150:126-31 (1993)). Overexpression of ErbB2 may lead to tumor growth via ligand-independent activation of ErbB2 or ErbB2 homodimers.

Antibodies directed against the rat p185neu and human ErbB2 protein products have been described. Drebin and colleagues have raised antibodies against the rat neu gene product, p185neu. See, for example, Drebin et al., Cell, 41:695-706 (1985); Myers et al., Meth. Enzym., 198:277-290 (1991); and WO94/22478. Drebin et al., Oncogene, 2:273-277 (1988) report that mixtures of antibodies reactive with two distinct regions of p185neu result in synergistic anti-tumor effects on neu-transformed NIH-3T3 cells implanted into nude mice. See also U.S. Patent 5,824,311 issued October 20, 1998.

Hudziak et al., Mol. Cell. Biol., 9(3):1165-1172 (1989) describe the generation of a panel of anti-ErbB2 antibodies which were characterized using the human breast tumor cell line SK-BR-3. Relative cell proliferation of the SK-BR-3 cells following exposure to the antibodies was determined by crystal violet staining of the monolayers after 72 hours. Using this assay, maximum inhibition was obtained with the antibody called 4D5 which inhibited cellular proliferation by 56%. Other antibodies in the panel reduced cellular proliferation to a lesser extent in this assay. The antibody 4D5 was further found to sensitize ErbB2-overexpressing breast tumor cell lines to the cytotoxic effects of TNF-α. See also U.S. Patent No. 5,677,171 issued October 14, 1997. The anti-ErbB2 antibodies discussed in Hudziak et al. are further characterized in Fendly et al., Cancer Research, 50:1550-1558 (1990); Kotts et al., In Vitro, 26(3):59A (1990); Sarup et al., Growth Regulation, 1:72-82 (1991); Shepard et al., J. Clin. Immunol., 11(3):117-127 (1991); Kumar et al., Mol. Cell. Biol., 11(2):979-986 (1991); Lewis et al., Cancer Immunol. Immunother., 37:255-263 (1993); Pietras et al., Oncogene, 9:1829-1838 (1994); Vitetta et al., Cancer Research, 54:5301-5309 (1994); Sliwkowski et al., J. Biol. Chem., 269 (20):14661-14665 (1994); Scott et al., J. Biol. Chem., 266:14300-5 (1991); D'souza et al., Proc. Natl. Acad Sci.; 91:7202-7206 (1994); Lewis et al., Cancer Research, 56:1457-1465 (1996); and Schaefer et al., Oncogene, 15:1385-1394 (1997).

A recombinant humanized version of the murine anti-ErbB2 antibody 4D5 (huMAb4D5-8, rhuMAb HER2 or HERCEPTIN^{®}; U.S. Patent No. 5,821,337) is clinically active in patients with ErbB2-overexpressing metastatic breast cancers that have received extensive prior anti-cancer therapy (Baselga et al., J. Clin Oncol., 14:737-744 (1996)). HERCEPTIN^{®} received marketing approval from the Food and Drug Administration September 25, 1998 for the treatment of patients with metastatic breast cancer whose tumors overexpress the ErbB2 protein. However, not all ErbB2 overexpressing tumors respond to HERCEPTIN^{®}. (Brockhoff et al., Cytometry, 44:338-48 (2001)). In addition, preclinical data suggest that HERCEPTIN^{®} may be therapeutically effective in treating non-small cell lung cancer (NSCLC). HER2 protein is overexprcssed in 20-66% of resected NSCLC tumors and has been shown to predict poor patient outcome in multiple series (Azzoli, C.G. et al., Semin. Oncol., 29(Suppl 4):59-65 (2002)).

Other anti-ErbB2 antibodies with various properties have been described in Tagliabue et al., Int. J. Cancer, 47:933-937 (1991); McKenzie et al., Oncogene, 4:543-548 (1989); Maier et al., Cancer Res., 51:5361-5369 (1991); Bacus et al., Molecular Carcinogenesis, 3:350-362 (1990); Stancovski et al., PNAS (USA), 88:8691-8695 (1991); Bacus et al., Cancer Research, 52:2580-2589 (1992); Xu et al., Int. J. Cancer, 53:401-408 (1993); WO94/00136; Kasprzyk et al., Cancer Research, 52:2771-2776 (1992);Hancock et al., Cancer Res., 51:4575-4580 (1991); Shawver et al, Cancer Res., 54:1367-1373 (1994); Arteaga et al., Cancer Res., 54:3758-3765 (1994); Harwerth et al., J. Biol. Chem., 267:15160-15167 (1992); U.S. Patent No. 5,783,186; and Klapper et al., Oncogene, 14:2099-2109 (1997). Monoclonal antibody 2C4 is described in WO 01/00245 2C4 has been shown to disrupt dimerization of HER2 with other ErbB receptor family members (WO 01/00245).

Homology screening has resulted in the identification of two other ErbB receptor family members; ErbB23 (U.S. Patent Nos. 5,183,884 and 5,480,968 as well as Kraus et al., PNAS (USA), 86:9193-9197 (1989)) and ErbB4 (EP Pat Appln No 599,274; Plowman et al., Proc. Natl. Acad. Sci. USA, 90:1746-1750 (1993); and Plowman et al., Nature, 366:473-475 (1993)), Both of these receptors display increased expression on at least some breast cancer cell lines.

The ErbB receptors are generally found in various combinations in cells and heterodimerization is thought to increase the diversity of cellular responses to a variety of ErbB ligands (Earp et al., Breast Cancer Research and Treatment, 35:115-132 (1995)). However, the mechanism by which these receptors aggregate and how this contributes to signaling is not fully understood (Brennan, P.J. et al., Oncogene, 19:6093-6101 (2000)). EGFR is bound by six different ligands; epidermal growth factor (EGF), transforming growth factor alpha (TGF-α), amphiregulin, heparin binding epidermal growth factor (HB-EGF), betacellulin and epiregulin (Groenen et al., Growth Factors, 11:235-257 (1994)). A family of heregulin proteins resulting from alternative splicing of a single gene are ligands for ErbB3 and ErbB4. The heregulin family includes alpha, beta and gamma heregulins (Holmes et al., Science, 256:1205-1210 (1992); U.S. Patent No. 5,641,869; and Schaefer et al., Oncogene, 15:1385-1394 (1997)); neu differentiation factors (NDFs), glial growth factors (GGFs); acetylcholine receptor inducing activity (ARIA); and sensory and motor neuron derived factor (SMDF). For a review, see Groenen et al., Growth Factors, 11:235-257 (1994); Lemke, G., Molec. & Cell. Neurosci., 7:247-262 (1996) and Lee et al., Pharm. Rev., 47:51-85 (1995). Recently three additional ErbB ligands were identified; neuregulin-2 (NRG-2) which is reported to bind either ErbB3 or ErbB4 (Chang et al., Nature, 387:509-512 (1997); and Carraway et al., Nature, 387:512-516 (1997)); neuregulin-3 which binds ErbB4 (Zhang et al., PNAS (USA), 94(18):9562-7 (1997)); and neuregulin-4 which binds ErbB4 (Harari et al., Oncogene, 18:2681-89 (1999)) HB-EGF, betacellulin and epiregulin also bind to ErbB4.

While EGF and TGFα do not bind ErbB2, EGF stimulates EGFR and ErbB2 to form a heterodimer, which activates EGFR and results in transphosphorylation of ErbB2 in the heterodimer. Dimerization and/or transphosphorylation appears to activate the ErbB2 tyrosine kinase. See Earp et al., supra. Likewise, heregulin does not bind ErbB2, but when ErbB3 is co-expressed with ErbB2, an active signaling complex is formed (Nagy et al., Cytometry, 32:120-31 (1998). Antibodies directed against ErbB2 are capable of disrupting this complex (Sliwkowski et al., J. Biol. Chem., 269(20):14661-14665 (1994)). ErbB3 is tyrosine kinase defective and thus requires heterodimerization, preferably with ErbB2, for signal transduction capabilities. (Graus-Porta et al., EMBO J., 16:1647-55 (1995)). Additionally, the affinity of ErbB3 for heregulin (HRG) is increased to a higher affinity state when co-expressed with ErbB2. See also, Levi et al., Journal of Neuroscience, 15:1329-1340 (1995); Morrissey et al., Proc. Natl. Acad. Sci. USA, 92:1431-1435 (1995); and Lewis et al., Cancer Res., 56:1457-1465 (1996) with respect to the ErbB2-ErbB3 protein complex. Indeed, ErbB2 is the preferred heterodimerization partner for both EGFR and ErbB3. (Graus-Porta et al., supra). ErbB4, like ErbB3, forms an active signaling complex with ErbB2 (Carraway and Cantley, Cell, 78:5-8 (1994)). Ligand-dependent heterodimerization of ErbB2 with EGFR or ErbB3 may promote the growth of tumors that express ErbB2.

The expression of the ErbB receptors and heregulin and the phosphorylation status of HER2 has been investigated in tumor specimens from primary breast cancer patients and in urinary bladder carcinoma (Esteva et al., Pathol. Oncol. Res., 7:171-177 (2001); Chow et al., Clin. Cancer Res., 7:1957-1962 (2001)). Correlation between active signaling through Her2/neu and clinicolathology and patient outcome in breast cancer has been reported by Thor et al., J. Clin. Oncology, 18:3230-3239 (2000), and DiGiovanna et aL, Cancer Res., 62:6667-6673 (2002).

Described herein is a method of identifying a tumor that is responsive to treatment with an anti-HER2 antibody, rhuMAb 2C4.

A sample of the tumor is obtained and the presence of a HER2/HER3 and/or HER2/HER1 and/or HER2/HER4 protein complex is detected in the sample. A tumor is identified as responsive to treatment with the anti-HER2 antibody when a complex is detected.

The presence of a complex may be detected by immunoprecipitating any protein complexes that comprise HER2 with an anti-HER2 antibody. The immunoprecipitated complexes are then contacted with an antibody selected from the group consisting of anti-HER3 antibodies, anti-HER1 antibodies, and anti-HER4 antibodies, and any binding is determined. A HER2/HER3 and/or HER2/HER1 and/or HER2/HER4 complex is detected if it is determined that anti-HER3 and/or anti-HER1 and/or anti-HER4 antibodies bind to the immunoprecipitated complexes.

The presence of a HER2/HER3 and/or HER2/HER1 and/or HER2/HER4 protein complex may be detected by contacting the tumor sample with an anti-HER2 antibody that comprises a first fluorophore. The tumor sample is then contacted with an antibody selected from the group consisting of anti-HER3 and/or anti-HER1 and/or anti-HER4 antibodies, wherein said antibody comprises a second fluorophore. Measurements of fluorescence resonance energy transfer are then made to determine if the first fluorophore and the second fluorophore are in close proximity. The presence of a HER2/HER3 and/or HER2/HER1 and/or HER2/HER4 protein complex is detected if the first and second fluorophore are determined to be in close proximity.

The presence of a HER2/HER3 and/or HER2/HER1 and/or HER2/HER4 complex may be detected by contacting the tumor sample with a first binding compound. The first binding compound comprises a first target binding moiety that specifically binds HER2. The first target binding moiety is preferably an anti-HER2 antibody or antibody fragment. The first binding compound further comprises a detectable moiety that is linked to the first binding domain by a cleavable linker.

The tumor sample is contacted with a second binding compound. The second binding compound preferably comprises a second target binding moiety that specifically binds HER3 or HER1 or HER4 and preferably does not bind HER2.

The second binding compound may bind HER3 or HER1, and not bind HER2 or HER4 or the second target binding moiety may comprise an anti-HER3 or anti-HER1 or anti-HER4 antibody or antibody fragment. The second binding compound is capable of cleaving the cleavable linker in the first binding compound upon activation, thus producing free detectable moiety if the first binding compound and the second binding compound are in close proximity. The presence of a HER2/HER3 or HER2/HER1 or HER2/HER4 protein complex is detected when the presence of the free detectable moiety is identified. The presence of the free detectable moiety may be identified by capillary electrophoresis.

The first binding compound may comprise a first target binding domain that specifically binds HER1 or HER3 or HER4, and the second binding compound may comprise a second target binding domain that specifically binds HER2.

The presence of a HER2/HER3 and/or HER2/HER1 and/or HER2/HER4 protein complex, and resultant HER2 activation, may be detected by assessing phosphorylation of ErbB receptor, for example by immunoprecipitation of the HER2 protein followed by Western blot immunodetection of phosphotyrosine.

The tumor sample that is analyzed for the presence of HER2/HER3 and/or HER2/HER1 and/or HER2/HER4 protein complexes is preferably obtained from a patient that is suffering from the tumor. The sample can, for example, be obtained by biopsy or by purifying circulating tumor cells from the patient or during surgery to remove the tumor from the patient.

The sample of the tumor may be obtained from a mammal other than the patient that originally developed the tumor. Preferably, the sample is obtained from a mouse, or another rodent. More preferably, the tumor is a xenografted tumor. The xenografted tumor is preferably produced by transplanting a fragment of a human tumor into a mouse, or another rodent

The tumor may be a lung tumor, more preferably a non-small cell lung cancer tumor. The tumor may be a mammary tumor.

Described herein is a method for identifying tumor cells as responsive to treatment with an antibody inhibiting the association of HER2 with another member of the ErbB receptor family, comprising the steps of (a) providing a biological sample comprising HER2-positive tumor cells; and (b) detecting the phosphorylation of an ErbB receptor in the biological sample, wherein said phosphorylation indicates that the tumor cells are responsive to treatment with the antibody. The phosphorylation of an ErbB2 (HER2) receptor may be detected.

Just as before, the other member associated with HER2 is HER3, HER1, and/or HER4, such as HER2 and/or HER1. The method can additionally comprise a step of detecting the presence of a HER2/HER3 and/or HER2/HER1 and/or HER2/HER4 protein complex, essentially as described above.

Described herein is a method for predicting the response of a subject diagnosed with a HER2-positive tumor to treatment with an antibody rhuMAb 2C4 inhibiting the association of HER2 with another member of the ErbB receptor family, by detecting the formation of HER2/HER3 and/or HER2/HER1 and/or HER2/HBR4 protein complexes and/or the phosphorylation of an ErbB receptor in a biological sample obtained from the subject, comprising HER2-positive tumor cells. The presence of such protein complexes and/or said phosphorylation indicates that the subject is likely to respond to treatment with the antibody. The detection of the phosphorylation of ErbB2 (HER2) receptor may indicate that the subject is likely to respond to treatment with the antibody.

Described herein is a method for identifying a subject responsive to treatment with an anti-HER2 antibody rhuMAb 2C4 by detecting phosphorylation of an ErbB receptor in circulating tumor cells of the subject The presence of such phosphorylation indicates that the subject is likely to respond to treatment with the anti-HER2 antibody. The ErbB2 (HER2) phosphorylation may be detected. The subject may be a human.

Also described is an article of manufacture comprising a container comprising an antibody rhuMAb 2C4 and instructions for administering the antibody to a patient suffering from a tumor. Preferably, the tumor has been determined to comprise HER2/ HER3 and/or HER2/HER1 and/or HER2/HER4 heterodimers.

### Summary of the Invention

The present invention is defined in the claims.

### Brief Description of the Drawings

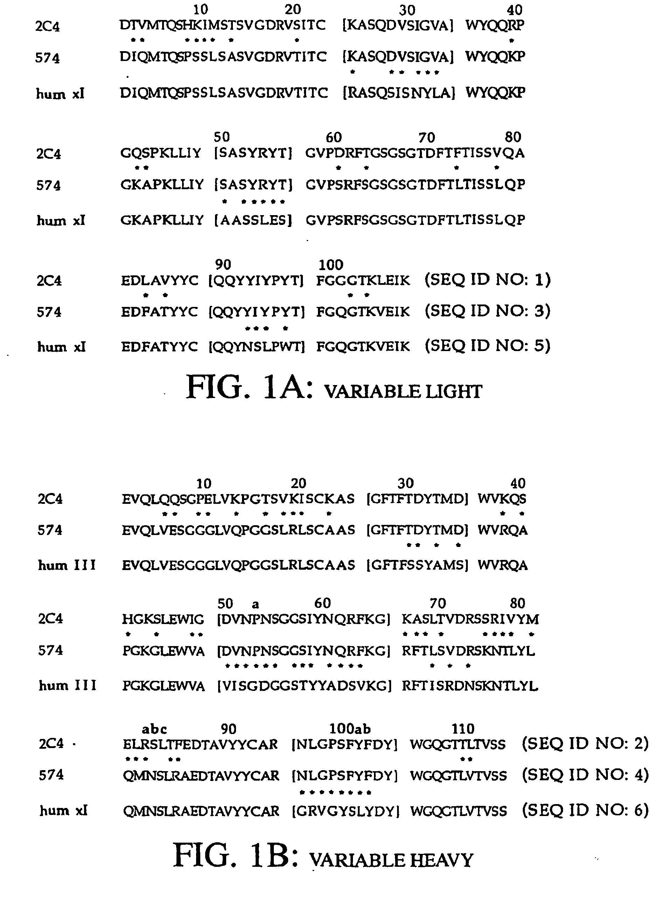

Figures 1A and 1B depict alignments of the amino acid sequences of the variable light (VL) (Fig. 1A) and variable heavy (VH) (Fig. 1B) domains of murine monoclonal antibody 2C4 (SEQ ID Nos. 1 and 2, respectively); VL and VH domains of humanized 2C4 version 574 (SEQ ID Nos. 3 and 4, respectively), and human VL and VH consensus frameworks (hum κ1, light kappa subgroup I; humIII, heavy subgroup III) (SEQ ID Nos. 5 and 6, respectively). Asterisks identify differences between humanized 2C4 version 574 and murine monoclonal antibody 2C4 or between humanized 2C4 version 574 and the human framework. Complementarity Determining Regions (CDRs) are in brackets.
Figures 2A and 2B show the effect of monoclonal antibody 2C4, HERCEPTIN^{®} antibody or an anti-EGFR antibody on heregulin (HRG) dependent association of ErbB2 with ErbB3 in MCF7 cells expressing low/normal levels of ErbB2 (Fig. 2A) and SK-BR-3 cells expressing high levels of ErbB2 (Fig. 2B); see Example 2 below.
Figure 3 is an immunoblot showing the presence of HER1/HER2 and HER2/HER3 heterodimers in protein extracts from non-small cell lung cancer xenograft explants.
Figure 4 is an immunoblot showing the presence of HER2 phosphorylation in protein extracts from non-small cell lung carcinoma (NSCLC) xenograph explants.

### Detailed Description of the Preferred Embodiment

Described herein is the experimental finding that responsiveness to the anti-HER2 antibody rhuMAb 2C4 correlates with the presence of HER2/HER3 and/or HER2/HER1 and or HBR2/HBR4 heterodimers, and/or the phosphorylation of an ErbB receptor in tumor cells. Thus, a tumor may be identified as responsive to treatment with the anti-HER2 antibody, based on the presence of HER2/HER3 and/or HER2/HER1 and/or HER2/HER4 heterodimers, and/or the phosphorylation of an ErbB receptor. HER2/HER3 and/or HER2/HER1 and/or HER2/HER4 heterodimers, and/or ErbB receptor phosphorylation may be identified by any method known in the art. By identifying specific tumors and tumor types that are responsive to treatment with anti-HER2 antibodies, patients can be identified who will likely benefit the most from such treatment. In addition, patients that would likely not benefit from therapy with monoclonal antibody 2C4 can be identified.

### Definitions

An "ErbB receptor" is a receptor protein tyrosine kinase which belongs to the ErbB receptor family and includes EGFR (ErbB1), ERRP, ErbB2, ErbB3 and ErbB4 receptors and other members of this family to be identified in the future. The ErbB receptor will generally comprise an extracellular domain, which may bind an ErbB ligand; a lipophilic transmembrane domain; a conserved intracellular tyrosine kinase domain; and a carboxyl-terminal signaling domain harboring several tyrosine residues which can be phosphorylated. The ErbB receptor may be a "native sequence" ErbB receptor or an "amino acid sequence variant" thereof. Preferably, the ErbB receptor is native sequence human ErbB receptor. Accordingly, a "member of the ErbB receptor family" is EGFR (ErbB1), ErbB2, ErbB3, ErbB4 or any other ErbB receptor currently known or to be identified in the future. Preferably, the member is EGFR (ErbB1), ErbB2, ErbB3, or ErbB4.

The terms "ErbB 1", "epidermal growth factor receptor", "EGFR" and "HER1" are used interchangeably herein and refer to EGFR as disclosed, for example, in Carpenter et al., Ann. Rev. Biochem., 56:881-914 (1987), including naturally occurring mutant forms thereof (e.g., a deletion mutant EGFR as in Humphrey et al., PNAS (USA), 87:4207-4211 (1990)). erbB1 refers to the gene encoding the EGFR protein product. Antibodies against HER1 are described, for example, in Murthy et al., Arch. Biochem. Biophys., 252:549-560 (1987) and in WO 95/25167.

The term "ERRP", "EGF-Receptor Related Protein", "EGFR Related Protein" and "epidermal growth factor receptor related protein" are used interchangeably herein and refer to ERRP as disclosed, for example in U.S. Patent No. 6,399,743 and U.S. Publication No. 2003/0096373.

The expressions "ErbB2" and "HER2" are used interchangeably herein and refer to human HER2 protein described, for example, in Semba et al., PNAS (USA), 82:6497-6501 (1985) and Yamamoto et al., Nature, 319:230-234 (1986) (Genebank accession number X03363). The term "erbB2" refers to the gene encoding human ErbB2 and "neu" refers to the gene encoding rat p 185neu. Preferred ErbB2 is native sequence human ErbB2.

"ErbB3" and "HER3" refer to the receptor polypeptide as disclosed, for example, in U.S. Patent Nos. 5,183,884 and 5,480,968 as well as Kraus et al., PNAS (USA), 86:9193-9197 (1989). Antibodies against ErbB3 are known in the art and are described, for example, in U.S. Patent Nos. 5,183,884, 5,480,968 and in WO 97/35885.

The terms "ErbB4" and "HER4" herein refer to the receptor polypeptide as disclosed, for example, in EP Pat Appln No 599,274; Plowman et al., Proc. Natl. Acad. Sci. USA, 90:1746-1750 (1993); and Plowman et al., Nature, 366:473-475 (1993), including isoforms thereof, e.g., as disclosed in WO 99/19488, published April 22, 1999. Antibodies against HER4 are described, for example, in WO 02/18444.

Antibodies to ErbB receptors are available commercially from a number of sources, including, for example, Santa Cruz Biotechnology, Inc., California, USA.

By "ErbB ligand" is meant a polypeptide which binds to and/or activates an ErbB receptor. The ErbB ligand may be a native sequence human ErbB ligand such as epidermal growth factor (EGF) (Savage et al., J. Biol. Chem., 247:7612-7621 (1972)); transforming growth factor alpha (TGF-α) (Marquardt et al., Science, 223:1079-1082 (1984)); amphiregulin also known as schwanoma or keratinocyte autocrine growth factor (Shoyab et al., Science, 243:1074-1076 (1989); Kimura et al., Nature, 348:257-260 (1990); and Cook et al., Mol. Cell. Biol., 11:2547-2557 (1991)); betacellulin (Shing et al., Science, 259:1604-1607 (1993); and Sasada et al., Biochem. Biophys. Res. Commun., 190:1173 (1993)); heparin-binding epidermal growth factor (HB-EGF) (Higashiyama et al., Science, 251:936-939 (1991)); epiregulin (Toyoda et al., J. Biol. Chem., 270:7495-7500 (1995); and Komurasaki et al., Oncogene, 15:2841-2848 (1997)); a heregulin (see below); neuregulin-2 (NRG-2) (Carraway et al., Nature, 387:512-516 (1997)); neuregulin-3 (NRG-3) (Zhang et al., Proc. Natl. Acad Sci., 94:9562-9567 (1997)); neuregulin-4 (NRG-4) (Harari et aL, Oncogene, 18:2681-89 (1999)) or cripto (CR-1) (Kannan et al., J. Biol. Chem., 272(6):3330-3335 (1997)). ErbB ligands which bind EGFR include EGF, TGF-α, amphiregulin, betacellulin, HB-EGF and epiregulin. ErbB ligands which bind ErbB3 include heregulins. ErbB ligands capable of binding ErbB4 include betacellulin, epiregulin, HB-EGF, NRG-2, NRG-3, NRG-4 and heregulins. The ErbB ligand may also be a synthetic ErbB ligand. The synthetic ligand may be specific for a particular ErbB receptor, or may recognize particular ErbB receptor complexes. An example of a synthetic ligand is the synthetic heregulin/egf chimera biregulin (see, for example, Jones et al., FEBS Letters, 447:227-231 (1999)).

"heregulin" (HRG) when used herein refers to a polypeptide encoded by the heregulin gene product as disclosed in U.S. Patent No. 5,641,869 or Marchionni et al., Nature, 362:312-318 (1993). Examples of heregulins include heregulin-α, heregulin-β1, heregulin-β2 and heregulin-β3 (Holmes et al., Science, 256:1205-1210 (1992); and U.S. Patent No. 5,641,869); neu differentiation factor (NDF) (Peles et al., Cell, 69: 205-216 (1992)); acetylcholine receptor-inducing activity (ARIA) (Falls et al., Cell, 72:801-815 (1993)); glial growth factors (GGFs) (Marchionni et at., Nature, 362:312-318 (1993)); sensory and motor neuron derived factor (SMDF) (Ho et al., J. Biol. Chem., 270:14523-14532 (1995)); γ-heregulin (Schaefer et al., Oncogene, 15:1385-1394 (1997)). The term includes biologically active fragments and/or amino acid sequence variants of a native sequence HRG polypeptide, such as an EGF-lke domain fragment thereof (e.g., HRGβ1177-244).

An "ErbB hetero-oligomer" herein is a noncovalently associated oligomer comprising at least two different ErbB receptors. An "ErbB dimer" is a noncovalently associated oligomer that comprises two different ErbB receptors. Such complexes may form when a cell expressing two or more ErbB receptors is exposed to an ErbB ligand. ErbB oligomers, such as ErbB dimers, can be isolated by immunoprecipitation and analyzed by SDS-PAGE as described in Sliwkowski et al., J. Biol. Chem., 269(20):14661-14665 (1994), for example. Examples of such ErbB hetero-oligomers include EGFR-ErbB2 (also referred to as HER1/HER2), ErbB2-ErbB3 (HBR2/HBR3) and ErbB3-ErbB4 (HER3/HER4) complexes. Moreover, the ErbB hetero-oligomer may comprise two or more ErbB2 receptors combined with a different ErbB receptor, such as ErbB3, ErbB4 or EGFR (ErbB1). Other proteins, such as a cytokine receptor subunit (*e.g*., gp130) may be included in the hetero-oligomer.

By "ligand activation of an ErbB receptor" is meant signal transduction (e.g., that caused by an intracellular kinase domain of an ErbB receptor phosphorylating tyrosine residues in the ErbB receptor or a substrate polypeptide) mediated by ErbB ligand binding to a ErbB hetero-oligomer comprising the ErbB receptor of interest. Generally, this will involve binding of an ErbB ligand to an ErbB hetero-oligomer which activates a kinase domain of one or more of the ErbB receptors in the hetero-oligomer and thereby results in phosphorylation of tyrosine residues in one or more of the ErbB receptors and/or phosphorylation of tyrosine residues in additional substrate polypeptides(s). ErbB receptor activation can be quantified using various tyrosine phosphorylation assays.

A "native sequence" polypeptide is one which has the same amino acid sequence as a polypeptide (*e.g*., ErbB receptor or ErbB ligand) derived from nature. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

The term "amino acid sequence variant" refers to polypeptides having amino acid sequences that differ to some extent from a native sequence polypeptide. Ordinarily, amino acid sequence variants will possess at least about 70% homology with at least one receptor binding domain of a native ErbB ligand or with at least one ligand binding domain of a native ErbB receptor, and preferably, they will be at least about 80%, more preferably, at least about 90% homologous with such receptor or ligand binding domains. The amino acid sequence variants possess substitutions, deletions, and/or insertions at certain positions within the amino acid sequence of the native amino acid sequence.

"Homology" is defined as the percentage of residues in the amino acid sequence variant that are identical after aligning the sequences and introducing gaps, if necessary, to achieve the maximum percent homology. Methods and computer programs for the alignment are well known in the art. One such computer program is "Align 2," authored by Genentech, Inc., which was filed with user documentation in the United States Copyright Office, Washington, DC 20559, on December 10, 1991.

The term "antibody" herein is used in the broadest sense and specifically covers intact monoclonal antibodies, polyclonal antibodies, multispecific antibodies (*e.g*., bispecific antibodies) formed from at least two intact antibodies, and antibody fragments, so long as they exhibit the desired biological activity.

The term "monoclonal antibody" as used herein refers to an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, *e.g*., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (*e.g*., Old World Monkey, Ape etc) and human constant region sequences.

"Antibody fragments" comprise a portion of an intact antibody, preferably comprising the antigen-binding or variable region thereof. Examples of antibody fragments include Fab, Fab', F(ab')2, and Fv fragments; diabodies; linear antibodies; single-chain antibody molecules; and multispecific antibodies formed from antibody fragment(s).

An "intact" antibody is one which comprises an antigen-binding variable region as well as a light chain constant domain (CL) and heavy chain constant domains, CH1, CH2 and CH3. The constant domains may be native sequence constant domains (*e.g*., human native sequence constant domains) or amino acid sequence variant thereof. Preferably, the intact antibody has one or more effector functions.

Antibody "effector functions" refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; down regulation of cell surface receptors (*e.g*., B cell receptor; BCR), etc.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), *e.g*., IgG1 IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Antibody-dependent cell-mediated cytotoxicity" and "ADCC" refer to a cell-mediated reaction in which nonspecific cytotoxic cells that express Fc receptors (FcRs) (*e.g*., Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol., 9:457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, *e.g*., in a animal model such as that disclosed in Clynes et al., PNAS (USA), 95:652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, *e.g*., from blood or PBMCs as described herein.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and Fcγ RIII subclasses, including allelic variants and alternatively spliced forms of these receptors. FcγRII receptors include Fc_{γ}RIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (See review M. in Daëron. Annu. Rev. Immunol., 15:203-234 (1997)). FcRs are reviewed in Ravetch and Kinet, Annu. Rev. Immunol., 9:457-92 (1991); Capel et al., Immunomethods, 4:25-34 (1994); and de Haas et al., J. Lab. Clin. Med., 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol., 117:587 (1976) and Kim et al., J. Immuno/., 24:249 (1994)).

"Complement dependent cytotoxicity" or "CDC" refers to the ability of a molecule to lyse a target in the presence of complement. The complement activation pathway is initiated by the binding of the first component of the complement system (C1q) to a molecule (*e.g.*, an antibody) complexed with a cognate antigen. To assess complement activation, a CDC assay, *e.g.,* as described in Gazzano-Santoro et al., J. Immunol. Methods, 202: 163 (1996), may be performed.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e.g., residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*e.g*., residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, J. Mol. Biol., 196:901-917 (1987)). "Framework Region" or "FR" residues are those variable domain residues other than the hypervariable region residues as herein defined.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')2 antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (X), based on the amino acid sequences of their constant domains.

"Single-chain Fv" or "scFv" antibody fragments comprise the VH and VL domains of antibody, wherein these domains are present in a single polypeptide chain. Preferably, the Fv polypeptide further comprises a polypeptide linker between the VH and VL domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994). Anti-ErbB2 antibody scFv fragments are described in WO 93/16185; U.S. Patent No. 5,571,894; and U.S. Patent No. 5,587,458.

The term "diabodies" refers to small antibody fragments with two antigen-binding sites, which fragments comprise a variable heavy domain (VH) connected to a variable light domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl, Acad Sci. USA, 90:6444-6448 (1993).

"humanized" forms of non-human (e.g., rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instance, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature, 321:522-525 (1986); Riechmann et al., Nature, 332:323-329 (1988); and Presta, Curr. Op. Struct. Biol., 2:593-596 (1992).

Humanized anti-ErbB2 antibodies include huMAb4D5-1, huMAb4D5-2, huMAb4D5-3, huMAb4D5-4, huMAb4D5-5, huMAb4D5-6, huMAb4D5-7 and huMAb4D5-8 (HERCEPTIN®) as described in Table 3 of U.S. Patent No. 5,821,337; humanized 520C9 (WO 93/21319) and humanized 2C4 antibodies as described herein below.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody "which binds" an antigen of interest, *e.g.*, ErbB2 antigen, is one capable of binding that antigen with sufficient affinity such that the antibody is useful in targeting a cell expressing the antigen. Where the antibody is one which binds ErbB2, it will usually preferentially bind ErbB2 as opposed to other ErbB receptors, and may be one which does not significantly cross-react with other proteins such as EGFR, ErbB3 or ErbB4. In such embodiments, the extent of binding of the antibody to these non-ErbB2 proteins (*e.g.,* cell surface binding to endogenous receptor) will be less than 10% as determined by fluorescence activated cell sorting (FACS) analysis or radioimmunoprecipitation (RIA). Sometimes, the anti-ErbB2 antibody will not significantly cross-react with the rat neu protein, *e.g*., as described in Schecter et al., Nature, 312:513 (1984) and Drebin et al., Nature, 312:545-548 (1984).

An antibody which "blocks" ligand activation of an ErbB receptor is one which reduces or prevents such activation as hereinabove defined, wherein the antibody is able to block ligand activation of the ErbB receptor substantially more effectively than monoclonal antibody 4D5, *e.g*., about as effectively as monoclonal antibodies 7F3 or 2C4 or Fab fragments thereof and preferably about as effectively as monoclonal antibody 2C4 or a Fab fragment thereof. For example, the antibody that blocks ligand activation of an ErbB receptor may be one which is about 50-100% more effective than 4D5 at blocking formation of an ErbB hetero-oligomer. Blocking of ligand activation of an ErbB receptor can occur by any means, *e.g.,* by interfering with: ligand binding to an ErbB receptor, ErbB complex formation, tyrosine kinase activity of an ErbB receptor in an ErbB complex and/or phosphorylation of tyrosine kinase residue(s) in or by an ErbB receptor. Examples of antibodies which block ligand activation of an ErbB receptor include monoclonal antibodies 2C4 and 7F3 (which block HRG activation of ErbB2/ErbB3 and ErbB2/ErbB4 hetero-oligomers; and EGF, TGF-α, amphiregulin, HB-EGF and/or epiregulin activation of an EGFR/ErbB2 hetero-oligomer); and L26, L96 and L288 antibodies (Klapper et al., Oncogene, 14:2099-2109 (1997)), which block EGF and NDF binding to T47D cells which express EGFR, ErbB2, ErbB3 and ErbB4.

An antibody having a "biological characteristic" of a designated antibody, such as the monoclonal antibody designated 2C4, is one which possesses one or more of the biological characteristics of that antibody which distinguish it from other antibodies that bind to the same antigen (*e.g.,* ErbB2). For example, an antibody with a biological characteristic of 2C4 may block HRG activation of an ErbB hetero-oligomer comprising ErbB2 and ErbB3, ErbB1 or ErbB4; block EGF, TGF-α, HB-EGF, epiregulin and/or amphiregulin activation of an ErbB receptor comprising EGFR and ErbB2; block EGF, TGF-α and/or HRG mediated activation of MAPK; and/or bind the same epitope in the extracellular domain of ErbB2 as that bound by 2C4 (*e.g.*, which blocks binding of monoclonal antibody 2C4 to ErbB2).

Unless indicated otherwise, the expression "monoclonal antibody 2C4" refers to an antibody that has antigen binding residues of, or derived from, the murine 2C4 antibody of the Examples below. For example, the monoclonal antibody 2C4 may be murine monoclonal antibody 2C4 or a variant thereof, such as humanized antibody 2C4, possessing antigen binding amino acid residues of murine monoclonal antibody 2C4. Examples of humanized 2C4 antibodies are provided herein and in WO 01/00245. Unless indicated otherwise, the expression "rhuMAb 2C4" when used herein refers to an antibody comprising the variable light (VL) and variable heavy (VH) sequences of SEQ ID Nos. 3 and 4, respectively, fused to human light and heavy IgG1 (non-A allotype) constant region sequences optionally expressed by a Chinese Hamster Ovary (CHO) cell.

Unless indicated otherwise, the term "monoclonal antibody 4D5" refers to an antibody that has antigen binding residues of, or derived from, the murine 4D5 antibody (ATCC CRL 10463). For example, the monoclonal antibody 4D5 may be murine monoclonal antibody 4D5 or a variant thereof, such as a humanized 4D5, possessing antigen binding residues of murine monoclonal antibody 4D5. Exemplary humanized 4D5 antibodies include huMAb4D5-1, huMAb4D5-2, huMAb4D5-3, huMAb4D5-4, huMAb4D5-5, huMAb4D5-6, huMAb4D5-7 and huMAb4D5-8 (HERCEPTIN®) as in US Patent No. 5,821,337, with huMAb4D5-8 (HERCEPTN®) being a preferred humanized 4D5 antibody.

A "growth inhibitory agent" when used herein refers to a compound or composition which inhibits growth of a cell, especially an ErbB expressing cancer cell either in vitro, or in vivo, Thus, the growth inhibitory agent may be one which significantly reduces the percentage of ErbB expressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

Examples of "growth inhibitory" antibodies are those which bind to ErbB2 and inhibit the growth of cancer cells overexpressing ErbB2. Preferred growth inhibitory anti-ErbB2 antibodies inhibit growth of SK-BR-3 breast tumor cells in cell culture by greater than 20%, and preferably greater than 50% (*e.g.,* from about 50% to about 100%) at an antibody concentration of about 0.5 to 30 µg/ml, where the growth inhibition is determined six days after exposure of the SK-BR-3 cells to the antibody (see U.S. Patent No. 5,677,171 issued October 14, 1997). The SK-BR-3 cell growth inhibition assay is described in more detail in that patent and herein below. The preferred growth inhibitory antibody is monoclonal antibody 4D5, *e.g.*, humanized 4D5.

An antibody which "induces cell death" is one which causes a viable cell to become nonviable. The cell is generally one which expresses the ErbB2 receptor, especially where the cell overexpresses the ErbB2 receptor. Preferably, the cell is a cancer cell, *e.g.*, a breast, ovarian, stomach, endometrial, salivary gland, lung, kidney, colon, thyroid, pancreatic or bladder cell. In vitro, the cell may be a SK-BR-3, BT474, Calu 3, MDA-MB-453, MDA-MB-361 or SKOV3 cell. Cell death in vitro may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (*i.e.,* in the absence of complement) and in the absence of immune effector cells. To determine whether the antibody is able to induce cell death, loss of membrane integrity as evaluated by uptake of propidium iodide (PI), trypan blue (see Moore et al., Cytotechnology, 17:1-11 (1995)) or 7AAD can be assessed relative to untreated cells. Preferred cell death-inducing antibodies are those which induce PI uptake in the PI uptake assay in BT474 cells (see below).

An antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses the ErbB2 receptor. Preferably, the cell is a tumor cell, *e.g.,* a breast, ovarian, stomach, endometrial, salivary gland, lung, kidney, colon; thyroid, pancreatic or bladder cell. In vitro, the cell may be a SK-BR-3, BT474, Calu 3 cell, MDA-MB-453, MDA-MB-361 or SKOV3 cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody which induces apoptosis is one which results in about 2 to 50 fold, preferably about 5 to 50 fold, and most preferably about 10 to 50 fold, induction of annexin binding relative to untreated cell in an annexin binding assay using BT474 cells (see below). Sometimes the pro-apoptotic antibody will be one which further blocks ErbB ligand activation of an ErbB receptor (*e.g.,* 7F3 antibody); i.e., the antibody shares a biological characteristic with monoclonal antibody 2C4. In other situations, the antibody is one which does not significantly block ErbB ligand activation of an ErbB receptor (*e.g.,* 7C2). Further, the antibody may be one like 7C2 which, while inducing apoptosis, does not induce a large reduction in the percent of cells in S phase (*e.g.,* one which only induces about 0-10% reduction in the percent of these cells relative to control).

The "epitope 2C4" is the region in the extracellular domain of ErbB2 to which the antibody 2C4 binds. In order to screen for antibodies which bind to the 2C4 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed to assess whether the antibody binds to the 2C4 epitope of ErbB2 (*e.g.,* any one or more residues in the region from about residue 22 to about residue 584 of ErbB2, inclusive; see Figs. 1A-B).

The "epitope 4D5" is the region in the extracellular domain of ErbB2 to which the antibody 4D5 (ATCC CRL 10463) binds. This epitope is close to the transmembrane domain of ErbB2. To screen for antibodies which bind to the 4D5 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed to assess whether the antibody binds to the 4D5 epitope of ErbB2 (*e.g.*, any one or more residues in the region from about residue 529 to about residue 625, inclusive; see Figs. 1A-B).

The "epitope 3H4" is the region in the extracellular domain of ErbB2 to which the antibody 3H4 binds. This epitope includes residues from about 541 to about 599, inclusive, in the amino acid sequence of ErbB2 extracellular domain; see Figs. 1A-B. The "epitope 7C2/7F3" is the region at the N terminus of the extracellular domain of ErbB2 to which the 7C2 and/or 7F3 antibodies (each deposited with the ATCC, see below) bind. To screen for antibodies which bind to the 7C2/7F3 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed to establish whether the antibody binds to the 7C2/7F3 epitope on ErbB2 (*e.g.*, any one or more of residues in the region from about residue 22 to about residue 53 of ErbB2; see Figs. 1A-B).

"Mammal" for purposes of treatment refers to any animal classified as a mammal, including humans, domestic and farm animals, and zoo, sports, or pet animals, such as dogs, horses, cats, cows, etc. Preferably, the mammal is human.

A tumor which is "responsive to treatment" shows statistically significant improvement in response to anti-ErbB antibody treatment when compared to no treatment or treatment with placebo in a recognized animal model or a human clinical trial, or which responds to initial treatment with anti-ErbB antibodies but grows as treatment is continued.

The terms "treat" or "treatment" refer to both therapeutic treatment and prophylactic or preventative measures, wherein the object is to prevent or slow down (lessen) an undesired physiological change or disorder, such as the development or spread of cancer. For purposes of this invention, beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

A "disorder" is any condition that would benefit from treatment of the present invention. This includes chronic and acute disorders or diseases including those pathological conditions which predispose the mammal to the disorder in question. Non-limiting examples of disorders to be treated herein include benign and malignant tumors; leukemias and lymphoid malignancies, in particular breast, ovarian, stomach, endometrial, salivary gland, lung, kidney, colon, thyroid, pancreatic, prostate or bladder cancer; neuronal, glial, astrocytal, hypothalamic and other glandular, macrophagal, epithelial, stromal and blastocoelic disorders; and inflammatory, angiogenic and immunologic disorders. A preferred disorder to be treated in accordance with the present invention is malignant tumor

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e.*, slow to some extent and preferably stop) cancer cell infiltration into peripheral organs; inhibit (*i.e*., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

The terms "cancer" and "cancerous" refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (*e.g.,* epithelial squamous cell cancer), lung cancer including small-cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

An "ErbB-expressing cancer" is one comprising cells which have ErbB protein present at their cell surface. An "ErbB2-expressing cancer" is one which produces sufficient levels of ErbB2 at the surface of cells thereof, such that an anti-ErbB2 antibody can bind thereto and have a therapeutic effect with respect to the cancer.

A cancer "characterized by excessive activation" of an ErbB receptor is one in which the extent of ErbB receptor activation in cancer cells significantly exceeds the level of activation of that receptor in non-cancerous cells of the same tissue type. Such excessive activation may result from overexpression of the ErbB receptor and/or greater than normal levels of an ErbB ligand available for activating the ErbB receptor in the cancer cells. Such excessive activation may cause and/or be caused by the malignant state of a cancer cell. In some embodiments, the cancer will be subjected to a diagnostic or prognostic assay to determine whether amplification and/or overexpression of an ErbB receptor is occurring which results in such excessive activation of the ErbB receptor. Alternatively, or additionally, the cancer may be subjected to a diagnostic or prognostic assay to determine whether amplification and/or overexpression an ErbB ligand is occurring in the cancer which attributes to excessive activation of the receptor. In a subset of such cancers, excessive activation of the receptor may result from an autocrine stimulatory pathway.

In an "autocrine" stimulatory pathway, self stimulation occurs by virtue of the cancer cell producing both an ErbB ligand and its cognate ErbB receptor. For example, the cancer may express or overexpress EGFR and also express or overexpress an EGFR ligand (*e.g.,* EGF, TGF-α, or HB-EGF). In another embodiment, the cancer may express or overexpress ErbB2 and also express or overexpress a heregulin (e.g. γ-HRG).

A cancer which "overexpresses" an ErbB receptor is one which has significantly higher levels of an ErbB receptor, such as ErbB2, at the cell surface thereof, compared to a noncancerous cell of the same tissue type. Such overexpression may be caused by gene amplification or by increased transcription or translation. ErbB receptor overexpression may be determined in a diagnostic or prognostic assay by evaluating increased levels of the ErbB protein present on the surface of a cell (*e.g.,* via an immunohistochemistry assay; IHC). Alternatively, or additionally, one may measure levels of ErbB-encoding nucleic acid in the cell, *e.g.,* via fluorescent in situ hybridization (FISH; see WO 98/45479 published October, 1998), southern blotting, or polymerase chain reaction (PCR) techniques, such as real time quantitative PCR (RT-PCR). Overexpression of the ErbB ligand, may be determined diagnostically by evaluating levels of the ligand (or nucleic acid encoding it) in the patient, *e.g.*, in a tumor biopsy or by various diagnostic assays such as the IHC, FISH, southern blotting, PCR or in vivo assays described above. One may also study ErbB receptor overexpression by measuring shed antigen (*e.g.,* ErbB extracellular domain) in a biological fluid such as serum (see, *e.g.,* U.S. Patent No. 4,933,294 issued June 12, 1990; WO 91/05264 published April 18, 1991; U.S. Patent No. 5,401,638 issued March 28, 1995; and Sias et al., J. Immunol. Methods, 132: 73-80 (1990)). Aside from the above assays, various other in vivo assays are available to the skilled practitioner. For example, one may expose cells within the body of the patient to an antibody which is optionally labeled with a detectable label, *e.g.,* a radioactive isotope, and binding of the antibody to cells in the patient can be evaluated, *e.g.,* by external scanning for radioactivity or by analyzing a biopsy taken from a patient previously exposed to the antibody.

The tumors overexpressing HER2 are rated by immunohistochemical scores corresponding to the number of copies of HER2 molecules expressed per cell, and can been determined biochemically: 0 = 0-10,000 copies/cell, 1+ = at least about 200,000 copies/cell, 2+ = at least about 500,000 copies/cell, 3+ = at least about 2,000,000 copies/cell. Overexpression of HER2 at the 3+ level, which leads to ligand-independent activation of the tyrosine kinase (Hudziak et al., Proc. Natl. Acad. Sci. USA, 84:7159-7163 [1987]), occurs in approximately 30% of breast cancers, and in these patients, relapse-free survival and overall survival are diminished (Slamon et al., Science, 244:707-712 [1989]; Slamon et al., Science, 235:177-182 [1987]).

Conversely, a cancer which is "not characterized by overexpression of the ErbB2 receptor" is one which, in a diagnostic assay, does not express higher than normal levels of ErbB2 receptor compared to a noncancerous cell of the same tissue type. A "hormone independent" cancer is one in which proliferation thereof is not dependent on the presence of a hormone which binds to a receptor expressed by cells in the cancer. Such cancers do not undergo clinical regression upon administration of pharmacological or surgical strategies that reduce the hormone concentration in or near the tumor. Examples of hormone independent cancers include androgen independent prostate cancer, estrogen independent breast cancer, endometrial cancer and ovarian cancer. Such cancers may begin as hormone dependent tumors and progress from a hormone-sensitive stage to a hormone-refractory tumor following anti-hormonal therapy.

The term "cytotoxic agent" as used herein refers to a substance that inhibits or prevents the function of cells and/or causes destruction of cells. The term is intended to include radioactive isotopes (*e.g.,* At211, I131, I125, Y90, Re186, Re188, Sm153, Bi212, P32 and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; rutrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C."); cyclophosphamide; thiotepa; taxanes, *e.g.*, paclitaxel (TAXOL®, Bristol-Myers Squibb Oncology, Princeton, NJ) and docetaxel (TAXOTERE®, Rh6ne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; esperamicins; capecitabine; and pharmaceutically acceptable salts, acids or derivatives of any of the above. Also included in this definition, are anti-hormonal agents that act to regulate or inhibit hormone action on tumors such as antiestrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

As used herein, the term "EGFR-targeted drug" refers to a therapeutic agent that binds to EGFR and, optionally, inhibits EGFR activation. Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, U.S. Patent No. 4,943, 533, Mendelsohn *et al*.) and variants thereof, such as chimerized 225 (C225 or Cetuximab; ERBUTIX®) and reshaped human 225 (H225) (see, WO 96/40210, Imclone Systems Inc.); antibodies that bind type II mutant EGFR (U.S. Patent No. 5,212,290); humanized and chimeric antibodies that bind EGFR as described in U.S. Patent No. 5,891,996; and human antibodies that bind EGFR, such as ABX-EGF (see WO 98/50433, Abgenix). The anti-EGFR antibody may be conjugated with a cyotoxic agent, thus generating an immunoconjugate (see, *e.g.*, EP 659,439A2, Merck Patent GmbH). Examples of small molecules that bind to EGFR include ZD1839 or Gefitinib (IRESSA™; Astra Zeneca), CP-358774 (TARCEVA™; Genentech/OSI) and AG1478, AG1571 (SU 5271; Sugen). A "tyrosine kinase inhibitor" is a molecule which inhibits to some extent tyrosine kinase activity of a tyrosine kinase such as an ErbB receptor. Examples of such inhibitors include the EGFR-targeted drugs noted in the preceding paragraph as well as quinazolines such as PD 153035,4-(3-chloroanilino) quinazoline, pyridopyrimidines, pyrimidopyrimidines, pyrrolopyrimidines, such as CGP 59326, CGP 60261 and CGP 62706, and pyrazolopyrimidines, 4-(phenylamino)-7H-pyrrolo[2,3-d] pyrimidines, curcumin (diferuloyl methane, 4,5-bis (4-fluoroanilino)phthalimide), tyrphostines containing nitrothiophene moieties; PD-0183805 (Warner-Lamber); antisense molecules (*e.g.*, those that bind to ErbB-encoding nucleic acid); quinoxalines (U.S. Patent No. 5,804,396); tryphostins (U.S. Patent No. 5,804,396); ZD6474 (Astra Zeneca); PTK-787 (Novartis/Schering AG); pan-ErbB inhibitors such as CI-1033 (Pfizer); Affinitac (ISIS 3521; Isis/Lilly); Imatinib mesylate (Gleevac; Novartis); PKI 166 (Novartis); GW2016 (Glaxo SmithKline); CI-1033 (Pfizer); EKB-569 (Wyeth); Semaxanib (Sugen); ZD6474 (AstraZeneca); PTK-787 (Novartis/Schering AG); INC-1C11 (Imclone); or as described in any of the following patent publications: U.S. Patent No. 5,804,396; WO 99/09016 (American Cyanimid); WO 98/43960 (American Cyanamid); WO 97/38983 (Warner Lambert); WO 99/06378 (Warner Lambert); WO 99/06396 (Warner Lambert); WO 96/30347 (Pfizer, Inc); WO 96/33978 (Zeneca); WO 96/3397 (Zeneca); and WO 96/33980 (Zeneca).

An "anti-angiogenic agent" refers to a compound which blocks, or interferes with to some degree, the development of blood vessels. The anti-angiogenic factor may, for instance, be a small molecule or antibody that binds to a growth factor or growth factor receptor involved in promoting angiogenesis. The preferred anti-angiogenic factor herein is an antibody that binds to Vascular Endothelial Growth Factor (VEGF).

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor, fibroblast growth factor, prolactin; placental lactogen; tumor necrosis factor-α and -β; mullerian-inhibiting substance; mouse gonadotropin-associated peptide; inhibin; activin; vascular endothelial growth factor, integrin; thrombopoietin (TPO); nerve growth factors such as NGF-β; platelet-growth factor, transforming growth factors (TGFs) such as TGF-α and TGF-β; insulin-like growth factor-I and -II; erythropoietin (EPO); osteoinductive factors; interferons such as interferon-α, -β, and -γ, colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. See, *e.g*., Wilman, "Prodrugs in Cancer Chemotherapy" Biochemical Society Transactions, 14, pp. 375-382, 615th Meeting Belfast (1986) and Stella et aL, "Prodrugs: A Chemical Approach to Targeted Drug Delivery," Directed Drug Delivery, Borchardt et al., (ed.), pp. 247-267, Humana Press (1985). Prodrugs include, but are not limited to, phosphate-containing prodrugs, thisophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form include, but are not limited to, those chemotherapeutic agents described above.

A "liposome" is a small vesicle composed of various types of lipids, phospholipids and/or surfactant which is useful for delivery of a drug (such as the anti-ErbB2 antibodies disclosed herein and, optionally, a chemotherapeutic agent) to a mammal. The components of the liposome are commonly arranged in a bilayer formation, similar to the lipid arrangement of biological membranes.

The term "package insert" is used to refer to instructions customarily included in commercial packages of therapeutic products, that contain information about the indications, usage, dosage, administration, contraindications and/or warnings concerning the use of such therapeutic products.

A "cardioprotectant" is a compound or composition which prevents or reduces myocardial dysfunction (*i.e*., cardiomyopathy and/or congestive heart failure) associated with administration of a drug, such as an anthracycline antibiotic and/or an anti-ErbB2 antibody, to a patient. The cardioprotectant may, for example, block or reduce a free-radical-mediated cardiotoxic effect and/or prevent or reduce oxidative-stress injury. Examples of cardioprotectants encompassed by the present definition include the iron-chelating agent dexrazoxane (ICRF-187) (Seifert et al., The Annals of pharmacotherapy, 28:1063-1072 (1994)); a lipid-lowering agent and/or anti-oxidant such as probucol (Singal et al., J. Mol Cell Cardiol., 27:1055-1063 (1995)); amifostine (aminothiol 2-[(3-aminopropyl)amino]ethanethiol-dihydrogen phosphate ester, also called WR-2721, and the dephosphorylated cellular uptake form thereof called WR-1065) and S-3-(3-methylaminopropylamino)propylphosphorothioic acid (WR-151327), see Green et aL, Cancer Research, 54:738-741 (1994); digoxin (Bristow, M.R. In: Bristow MR, ed. Drug-Induced Heart Disease. New York: Elsevier 191-215 (1980)); beta-blockers such as metoprolol (Hjalmarson et al., Drugs 47:Suppl4:31-9 (1994); and Shaddy et al., Am. Heart J., 129:197-9 (1995)); vitamin E; ascorbic acid (vitamin C); free radical scavengers such as oleanolic acid, ursolic acid and N-acetylcysteine (NAC); spin trapping compounds such as alpha-phenyl-tert-butyl nitrone (PBN); (Paracchini et al., Anticancer Res., 13:1607-1612 (1993)); selenoorganic compounds such as P251 (Elbesen); and the like.

An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

### Methods of Identifying Tumors that are Responsive to Treatment with Anti-HER2 Antibodies

### Sources of Tumors and Tumor Cells

Tumors can be characterized as responsive to therapy with 2CA , based on the presence of EGFR ErbB2 and/or ErbB2-ErbB3 heterodimers on the cell surface, as a measure of HER2 activation. Tumor samples may be assayed for the presence of heterodimers by any method known in the art Preferably, the presence of heterodimers is determined by one or more of the methods described below.

Since HER2 activation is the result of receptor heterodimerization and phosphorylation, a particularly important method for identifying tumors responsive to therapy with 2C4 is the detection of phosphorylation ofErbB receptor, such as phosphorylation of ErbB2 (HER2) receptor, as described below.

Sources of tumor cells that may be assayed include, but are not limited to, tumor biopsies, circulating tumor cells, circulating plasma proteins, ascitic fluid, xenotransplanted tumors and other tumor models, and primary cell cultures or cell lines derived from tumors or exhibiting tumor-like properties, as well as preserved tumor samples, such as formalin-fixed, paraffin-embedded tumor samples. The screening of panels of various tumor cell types for EGFR-ErbB2 and/or ErbB2-BrbB3 heterodimers, and/or phosphorylation of ErbB receptor is contemplated by the present invention. Tumor cells of the same type as tumor cells that test positive for heterodimers, and/or phosphorylation of ErbB receptor, such as ErbB2 (HER2) receptor, may be subjected to therapy with 2C4. The tumor models described below are provided as examples and should not be construed as limiting the invention.

Tumor cells that originate with a patient currently suffering from a tumor may be assayed for responsiveness to therapy with 2C4. If the cells are determined to be responsive, based on the presence of HER2/HER3 and/or HBR2/HBR1 heterodimers or by demonstrating the phosphorylation of ErbB receptor, the patient may subsequently be treated with an antibody with one or more of the biological characteristics of 2C4. The patient is treated with rhuMAb 2C4.

Tumor cells from particular type of tumor or cells that are believed to have the characteristics of a particular type of tumor may be assayed for the presence of EGFR-ErbB2 and/or ErbB2-ErbB3 heterodimers, or for the phosphorylation of ErbB receptor, preferable ErbB2 (HER2) receptor. If EGFR-ErbB2 and/or ErbB2-ErbB3 heterodimers and/or phosphorylation of ErbB receptor is detected, that type of tumor is considered to be a good candidate for treatment with rhuMAP 2C4. Patients suffering from that type of tumor may then be treated with such an antibody.

### Cell Line Xenografts

In vitro propagated tumor cells, such as tumor cells grown in culture and tumor cell lines, may be xenografted into mice by implanting cells directly into a site of interest. Such methods are well known to one of skill in the art. The cells are assayed to identify the presence of EGFR-ErbB2 and/or ErbB2-ErbB3 heterodimers, or for the phosphorylation of ErbB receptor, such as the phosphorylation of ErbB2 (HER2) receptor.

Tumor cells may be implanted subcutaneously into a mouse, preferably an athymic nude mouse. Tumor cells may be implanted into a physiologically relevant location to create an appropriate in situ tumor model. For example, cells from a breast cancer cell line may be implanted at various concentrations into the mammary fat pad of athymic nude mice to more accurately model the biology of breast cancer. Tumor cells may be assayed for the presence of EGFR ErbB2 or ErbB2-ErbB3 heterodimers, or for the phosphorylation of ErbB receptor either before or after implantation. Preferably, tumor cells are assayed after the implanted cells have developed into a tumor of a predetermined size. The mice may also be subjected to therapy with 2C4 or a functionally equivalent antibody, with untreated mice serving as a control.

Similar models may be established for any type of tumor from which cultured cells or cell lines have been derived. Tumor types include, but are not limited to, bladder, brain, breast, colon, esophagus, kidney, leukemia, liver, lung, melanoma, ovary, pancreas, prostate, sarcoma, stomach, testicle, and uterus. Tumor cells or cell lines that overexpress ErbB2 are used for implantation, or tumor cells or cell lines that express normal or below normal amounts of ErbB2 may be used for implantation. Tumor cells or cell lines non-responsive to HERCEPTIN® may be used for implantation.

Approximately 20 million MDA-175 breast tumor cells may be implanted into the mouse gonadal fat pad. Expression of HER2/HBR1 and/or HER2/HER3 dimers on the surface of xenografted cells is determined, such as by one of the methods described below. Mice thus implanted may also be subjected to treatment with 0, 3 mg/kg, 10 mg/kg, 30 mg/kg, or 100 mg/kg 2C4. Other dosage regimens would be within the determination of one of ordinary skill in the art.

Solid tumors, like breast cancer, ovarian cancer, lung cancer, prostate cancer and colorectal cancer, are particularly suitable for analysis and treatment.

### Tumor Xenografts

Mammalian tumor specimens, preferably human tumor specimens, may be obtained and implanted into mice, preferably athymic nude mice. The tumor specimens may be obtained by any method known in the art. The tumor specimens may be surgically resected, such as in a biopsy or in the process of surgery to remove the tumor from the mammal. The tumor specimen may be obtained by purifying circulating tumor cells from the mammals blood.

Solid human tumor slices of approximately 5 x5 x0.5 to 1mm in diameter may be implanted into the flanks of athymic nude mice, generally four fragments per mouse. When the implanted tumors reach a median diameter of about 10-15 mm, they may be serially passaged, generally using smaller tumor fragments. Methods of generating and studying human tumor xenografts are described in the following references: Fiebig et al., "Human Tumor Xenografts: Predictivity, Characterization and Discovery of New Anticancer Agents," in Contributions to Oncology: Relevance of Tumor Models for Anticancer Drug Development, Fiebig & Burger, eds. (Basel, Karger1999), vol. 54, pp. 29-50; Berger et al., "Establishment and Characterization of Human Tumor Xenografts in Thymus-Aplastic Nude Mice," in Immunodeficient Mice in Oncology, Fiebig & Berger, eds. (Basel, Karger 1992), pp. 23-46; Fiebig & Burger, "Human Tumor Xenografts and Explants," in Models in Cancer Research Teicher, ed. (Humana Press 2002) pp. 113-137.

Human xenografts are considered highly predictive of tumor behavior within the donor patient, as the xenograft grows as a solid tumor, differentiates, and develops a stroma, vasculature, and a central necrosis. In most cases, xenografts retain most of the molecular, histological, and pathophysiological characteristics of the fresh patient-derived tumor. Tumor cells from mice containing first or serially passaged tumors may be analyzed for the presence of EGFR-ErbB2 and/or ErbB2-ErbB3 heterodimers, or for the phosphorylation of ErbB receptor. Mice may also be subjected to therapy with 2C4 or a functionally equivalent antibody.

A newly created or established panel of human tumor xenografts may be screened for the presence of EGFR-ErbB2 or ErbB2-ErbB3 heterodimers, or for phosphorylation of ErbB receptor. Fiebig & Burger, supra, describe a panel of over 300 human tumor xenografts established from a variety of common cancer types, such as bladder, brain, breast, colon, esophagus, kidney, leukemia, liver, lung, melanoma, ovary, pancreas, prostate, sarcoma, stomach, testicle, and uterus. The entire panel may be screened for heterodimers, or for phosphorylation of ErbB receptor, such as ErbB2 (HER2) receptor. Subsets of this panel may also be screened for heterodimers., or for phosphorylation ofErbB receptor, wherein subsets are based on, for example, tissue type, over-, under-, or normal expression of ErbB2, or failure to respond to HERCEPTIN®. In this manner, tumors may be categorized as candidates for therapy with 2C4 based on the presence of heterodimers, or by demonstrating the phosphorylation of ErbB receptor, such as ErbB2 (HBR2) receptor. Likewise, patients possessing tumors thus categorized may be more rapidly deemed eligible for therapy with 2C4 or a functionally equivalent antibody.

Tumor specimens may be assayed for the presence of EGFR-ErbB2 or ErbB2-BrbB3 heterodimers, or for the phosphorylation of ErbB receptor either before or after implantation. Approximately one gram of tumor from a first and/or serially passaged xenograft may be further characterized molecularly for heterodimers or snap frozen in liquid nitrogen and stored at -80°C for later characterization. Xenograft tumors may be further analyzed by a double layer soft-agarassay, also called a clonogenic assay, as described, for example, in Fiebig & Burger, supra. Solid human tumor xenografts are mechanically and proteolytically disaggregated into a single-cell suspension, which is plated into multiwell plates layered with soft agar as described. Tumor cell growth in vitro leads to the formation of colonies, which may be further analyzed for molecular characteristics, such as heterodimers, or for phosphorylation of ErbB receptor, or for other histochemical or morphological characteristics.

### B. Detection of EGFR-ErbB2 and ErbB2-ErbB3 Heterodimers

Any method known in the art may be used to detect EGFR ErbB2 or ErbB2-ErbB3 heterodimers in tumors. Several preferred methods are described below. These methods detect noncovalent protein-protein interactions or otherwise indicate proximity between proteins of interest. The methods described below are provided as examples and should not be construed as limiting the invention.

### Co-Immunoprecipitadon and Immunoblotting

Immunoaffinity-based methods, such as immunoprecipitation or ELISA, may be used to detect EGFR-ErbB2 or ErbB2-ErbB3 heterodimers. Anti-ErbB2 antibodies may be used to immunoprecipitate complexes comprising ErbB2 from tumor cells, and the resulting immunoprecipitant is then probed for the presence of EGFR or ErbB3 by immunoblotting. EGFR or ErbB3 antibodies may be used for the immunoprecipitation step and the immunoprecipitant then probed with ErbB2 antibodies. ErbB ligands specific to HBR1, HER3, HER2/HER1 complexes or HER2/HER3 complexes may be used to precipitate complexes, which are then probed for the presence of HER2. For example, ligands may be conjugated to avidin and complexes purified on a biotin column.

In other assays such as ELISA or antibody "sandwich"-type assays, antibodies to ErbB2 are immobilized on a solid support, contacted with tumor cells or tumor cell lysate, washed, and then exposed to antibody against EGFR or ErbH3. Binding of the latter antibody, which may be detected directly or by a secondary antibody conjugated to a detectable label, indicates the presence of heterodimers. EGFR or ErbB3 antibody may be immobilized, and ErbB2 antibody is used for the detection step. ErbB receptor ligands may be used in place of, or in combination with anti-ErbB receptor antibodies.

Immunoprecipitation with EGFR, ErbB2, or ErbB3 antibody may be followed by a functional assay for heterodimers, as an alternative or supplement to immunoblotting. Immunoprecipitation with ErbB3 antibody may be followed by an assay for receptor tyrosine kinase activity in the immunoprecipitant Because ErbB3 does not have intrinsic tyrosine kinase activity, the presence of tyrosine kinase activity in the immunoprecipitant indicates that ErbB3 is most likely associated with ErbB2. Graus-Porta et al., EMBO J., 16:1647-55 (1997); Klapper et al., Proc. Natl. Acad Sci. USA, 96:4995-5000 (1999). This result may be confirmed by immunoblotting with ErbB2 antibodies. Immunoprecipiation with ErbB2 antibody may be followed by an assay for EGFR receptor tyrosine kinase activity. In this assay, the immunoprecipitant is contacted with radioactive ATP and a peptide substrate that mimics the in vivo site of transphosphorylation of ErbB2 by EGFR. Phosphorylation of the peptide indicates co-immunoprecipitation and thus heterodimerization of EGFR with ErbB2. Receptor tyrosine kinase activity assays are well known in the art and include assays that detect phosphorylation of target substrates, for example, by phosphotyrosine antibody, and activation of cognate signal transduction pathways, such as the MAPK pathway.

Variations on the above methods and assays would be readily apparent and routine to one of ordinary skill in the art.

Chemical or UV cross-linking may also be used to covalently join heterodimers on the surface of living cells. Hunter et al., Biochem. J., 320:847-53. Examples of chemical cross-linkers include dithiobis(succinimidyl) propionate (DSP) and 3,3'dithiobis(sulphosuccinimidyl) propionate (DTSSP). Cell extracts from chemically cross-linked tumor cells may be analyzed by SDS-PAGE and immunoblotted with antibodies to EGFR and/or ErbB3. A supershifted band of the appropriate molecular weight most likely represents EGFR-ErbB2 or ErbB2-ErbB3 heterodimers, as ErbB2 is the preferred heterodimerization partner for EGFR and ErbB3. This result may be confirmed by subsequent immunoblotting with ErbB2 antibodies.

### FRET and fluorescence-Based Methods

Fluorescence resonance energy transfer (FRET) may also be used to detect EGFR-ErbB2 or ErbB2-ErbB3 heterodimers. FRET detects protein conformational changes and protein-protein interactions in vivo and in vitro based on the transfer of energy from a donor fluorophore to an acceptor fluorophore. Selvin, Nat. Struct. Biol., 7:730-34 (2000). Energy transfer takes place only if the donor fluorophore is in sufficient proximity to the acceptor fluorophore. In a typical FRET experiment, two proteins or two sites on a single protein are labeled with different fluorescent probes. One of the probes, the donor probe, is excited to a higher energy state by incident light of a specified wavelength. The donor probe then transmits its energy to the second probe, the acceptor probe, resulting in a reduction in the donor's fluorescence intensity and an increase in the acceptor's fluorescence emission. To measure the extent of energy transfer, the donor's intensity in a sample labeled with donor and acceptor probes is compared with its intensity in a sample labeled with donor probe only. Optionally, acceptor intensity is compared in donor/acceptor and acceptor only samples. Suitable probes are known in the art and include, for example, membrane permeant dyes, such as fluorescein and rhodamine, organic dyes, such as the cyanine dyes, and lanthanide atoms. Selvin, supra. Methods and instrumentation for detecting and measuring energy transfer are also know in the art. Selvin, supra.

FRET-based techniques suitable for detecting and measuring protein-protein interactions in individual cells are also known in the art. For example, donor photobleaching fluorescence resonance energy transfer (pbFRET) microscopy and fluorescence lifetime imaging microscopy (FLIM) may be used to detect the dimerization of cell surface receptors. Selvin, supra; Gadella & Jovin, J. Cell Biol., 129:1543-58 (1995). pbFRET may be used on cells either "in suspension" or "in situ" to detect and measure the formation of EGFR-ErbB2 or ErbB2-BrbB3 heterodimers, as described in Nagy et al., Cytometry, 32:120-131 (1998). These techniques measure the reduction in a donor's fluorescence lifetime due to energy transfer.

A flow cytometric Foerster-type FRET technique (FCET) may be used to investigate EGFR-ErbB2 and ErbB2-ErbB3 heterodimerization, as described in Nagy et al., supra, and Brockhoff et al., Cytometry, 44:338-48 (2001).

FRET is preferably used in conjunction with standard immunohistochemical labeling techniques. Kenworthy, Methods, 24:289-96 (2001). For example, antibodies conjugated to suitable fluorescent dyes can be used as probes for labeling two different proteins. If the proteins are within proximity of one another, the fluorescent dyes act as donors and acceptors for FRET. Energy transfer is detected by standard means. Energy transfer may be detected by flow cytometric means or by digital microscopy systems, such as confocal microscopy or wide-field fluorescence microscopy coupled to a charge-coupled device (CCD) camera.

ErbB2 antibodies and either EGFR or ErbB3 antibodies may be directly labeled with two different fluorophores, for example as described in Nagy et al, supra. Tumor cells or tumor cell lysates are contacted with the differentially labeled antibodies, which act as donors and acceptors for FRET in the presence of EGFR-ErbB2 or ErbB2-ErbB3 heterodimers. Alternatively, unlabeled antibodies against BrbB2 and either EGFR or ErbB3 are used along with differentially labeled secondary antibodies that serve as donors and acceptors. See, for example, Brockhoff et al., supra. Energy transfer is detected and the presence of heterodimers is determined if the labels are found to be in close proximity.

ErbB receptor ligands that are specific for HER2 and either HER1 HER3 may be fluorescently labeled and used for FRET studies.

The presence of heterodimers on the surface of tumor cells is demonstrated by co-localization of ErbB2 with either EGFR or ErbB3 using standard direct or indirect immunofluorescence techniques and confocal laser scanning microscopy. Alternatively, laser scanning imaging (LSI) is used to detect antibody binding and co-localization of ErbB2 with either EGFR or ErbB3 in a high-throughput format, such as a microwell plate, as described in Zuck et al, Proc. Natl. Acad Sci. USA. 96:11122-27 (1999).

The presence of EGFR-ErbB2 and/or ErbB2-ErbB3 heterodimers may be determined by identifying enzymatic activity that is dependent upon the proximity of the heterodimer components. A ErbB2 antibody is conjugated with one enzyme and an EGFR or ErbB3 antibody is conjugated with a second enzyme. A first substrate for the first enzyme is added and the reaction produces a second substrate for the second enzyme. This leads to a reaction with another molecule to produce a detectable compound, such as a dye. The presence of another chemical breaks down the second substrate, so that reaction with the second enzyme is prevented unless the first and second enzymes, and thus the two antibodies, are in close proximity. Tumor cells or cell lysates may be contacted with an ErbB2 antibody that is conjugated with glucose oxidase and an ErbB3 or ErbB1 antibody that is conjugated with horse radish peroxidase. Glucose is added to the reaction, along with a dye precursor, such as DAB, and catalase. The presence of heterodimers is determined by the development of color upon staining for DAB.

### eTag™ Assay System

Heterodimers may be detected using methods based on the eTag™ assay system (Aclara Bio Sciences, Mountain View, CA), as described, for example, WO 83502 and in U.S. Patent Application 2001/0049105, published December 6, 2001,

An eTag™, or "electrophoretic tag," comprises a detectable reporter moiety, such as a fluorescent group. It may also comprise a "mobility modifier," which consists essentially of a moiety having a unique electrophoretic mobility. These moieties allow for separation and detection of the eTag™ from a complex mixture under defined electrophoretic conditions, such as capillary electrophoresis (CE). The portion of the eTag™ containing the reporter moiety and, optionally, the mobility modifier is linked to a first target binding moiety by a cleavable linking group to produce a first binding compound. The first target binding moiety specifically recognizes a particular first target, such as a nucleic acid or protein. The first target binding moiety is not limited in any way, and may be for example, a polynucleotide or a polypeptide. Preferably, the first target binding moiety is an antibody or antibody fragment. Alternatively, the first target binding moiety may be an ErbB receptor ligand or binding-competent fragment thereof.

The linking group preferably comprises a cleavable moiety, such as an enzyme substrate, or any chemical bond that may be cleaved under defined conditions. When the first target binding moiety binds to its target, the cleaving agent is introduced and/or activated, and the linking group is cleaved, thus releasing the portion of the eTag™ containing the reporter moiety and mobility modifier. Thus, the presence of a "free" eTag™ indicates the binding of the target binding moiety to its target.

Preferably, a second binding compound comprises the cleaving agent and a second target binding moiety that specifically recognizes a second target. The second target binding moiety is also not limited in any way and may be, for example, an antibody or antibody fragment or an ErbB receptor ligand or binding competent ligand fragment. The cleaving agent is such that it will only cleave the linking group in the first binding compound if the first binding compound and the second binding compound are in close proximity.

A first binding compound may comprise an eTag™ in which an antibody to ErbB2 serves as the first target binding moiety. A second binding compound may comprise an antibody to EGFR or ErbB3 joined to a cleaving agent capable of cleaving the linking group of the eTag™. Preferably the cleaving agent must be activated in order to be able to cleave the linking group. Tumor cells or tumor cell lysates are contacted with the eTag™, which binds to ErbB2, and with the modified EGFR or ErbB3 antibody, which binds to EGFR or ErbB3 on the cell surface. Unbound binding compound is preferable removed, and the cleaving agent is activated, if necessary. If EGFR-ErbB2 or ErbB2-ErbB3 heterodimers are present, the cleaving agent will cleave the linking group and release the eTag™ due to the proximity of the cleaving agent to the linking group. Free eTag™ may then be detected by any method known in the art, such as capillary electrophoresis.

The cleaving agent may be an activatable chemical species that acts on the linking group. For example, the cleaving agent may be activated by exposing the sample to light.

The eTag™ may be constructed using an antibody to EGFR or ErbB3 as the first target binding moiety, and the second binding compound is constructed from an antibody to ErbB2.

### Detection of Phosphorylation of ErbB Receptor

The presence of the phosphorylation of ErbB receptor may be used to demonstrate HER2 activation.

Phosphorylation of ErbB receptor may be assessed by immunoprecipitation of one or more ErbB receptors, such as ErbB2 (HER2) receptor, and Western blot analysis. For example, positivity is determined by the presence of a phospho-HER2 band on the gel, using an anti-phosphotyrosine antibody to detect phosphorylated tyrosine residue(s) in the immunoprecipitated ErbB receptor(s). Anti-phosphotyrosine antibodies are commercially available from PanVera (Madison, WI), a subsidiary of Invitrogen, Chemicon International Inc. (Temecula, CA), or Upstate Biotechnology (Lake Placid, NY). Negativity is determined by the absence of the band.

Phosphorylation of ErbB2 (HER2) receptor may be assessed by immunohistochemistry using a phospho-specific anti-HER2 antibody (clone PN2A; Thor et al, J. Clin. Oncol., 18(18):3230-3239 (2000)).

Other methods for detecting phosphorylation of ErbB receptor(s) include, but are not limited to, KIRA ELISA (U.S. Patent Nos. 5,766,863; 5,891,650; 5,914,237; 6,025,145; and 6,287,784), mass spectrometry (comparing size of phosphorylated and non-phosphorylated HER2), and e-tag proximity assay with anti-HER2 antibody (*e.g*., using the eTag™ assay kit available from Aclara BioSciences (Mountain View, CA). Details of the eTag™ assay are described hereinabove.

### Vectors, Host Cells and Recombinant Methods

Described herein are isolated nucleic acid encoding the humanized anti-ErbB2 antibody rhu MAb 2C4 vectors and host cells comprising the nucleic acid, and recombinant techniques for the production of the antibody. For recombinant production of an antibody, the nucleic acid encoding it is isolated and inserted into a replicable vector for further cloning (amplification of the DNA) or for expression. DNA encoding the monoclonal antibody is readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence.

### Signal sequence component

Rhu MAb 2C4 may be produced recombinantly not only directly, but also as fusion polypeptides with a heterologous polypeptide, which is preferably a signal sequence or other polypeptide having a specific cleavage site at the N-terminus of the mature protein or polypeptide. The heterologous signal sequence selected preferably is one that is recognized and processed (*i.e*., cleaved by a signal peptidase) by the host cell. For prokaryotic host cells that do not recognize and process the native anti-ErbB2 antibody signal sequence, the signal sequence is substituted by a prokaryotic signal sequence selected, for example, from the group of the alkaline phosphatase, penicillinase, lpp, or heat-stable enterotoxin II leaders. For yeast secretion the native signal sequence may be substituted by, *e.g.,* the yeast invertase leader, α factor leader (including Saccharomyces and Kluyveromyces α-factor leaders), or acid phosphatase leader, the C. albicans glucoamylase leader, or the signal described in WO 90/13646. In mammalian cell expression, mammalian signal sequences as well as viral secretory leaders, for example, the herpes simplex gD signal, are available.

The DNA for such precursor region is ligated in reading frame to DNA encoding the anti-ErbB2 antibody.

### Origin of replication component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

### Selection gene component

Expression and cloning vectors may contain a selection gene, also termed a selectable marker. Typical selection genes encode proteins that (a) confer resistance to antibiotics or other toxins, *e.g*., ampicillin, neomycin, methotrexate, or tetracycline, (b) complement auxotrophic deficiencies, or (c) supply critical nutrients not available from complex media, *e.g.,* the gene encoding D-alanine racemase for Bacilli.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the anti-ErbB2 antibody nucleic acid, such as DHFR, thymidine kinase, metallothionein-I and -II, preferably primate metallothionein genes, adenosine deaminase, ornithine decarboxylase, etc.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding anti-ErbB2 antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Patent No. 4,965,199.

A suitable selection gene for use in yeast is the trp1 gene present in the yeast plasmid YRp7 (Stinchcomb et al., Nature, 282:39 (1979)). The trp1 gene provides a selection marker for a mutant strain of yeast lacking the ability to grow in tryptophan, for example, ATCC No. 44076 or PEP4-1. Jones, Genetics, 85:12 (1977). The presence of the trp1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the Leu2 gene.

In addition, vectors derived from the 1.6 µm circular plasmid pKD1 can be used for transformation of Kluyveromyces yeasts. Alternatively, an expression system for large-scale production of recombinant calf chymosin was reported for K. lactis. Van den Berg, Bio/Technology, 8:135 (1990). Stable multi-copy expression vectors for secretion of mature recombinant human serum albumin by industrial strains of Kluyveromyces have also been disclosed. Fleer et al., Bio/Technology, 9:968-975 (1991).

### Promoter component

Expression and cloning vectors usually contain a promoter that is recognized by the host organism and is operably linked to the anti-ErbB2 antibody nucleic acid. Promoters suitable for use with prokaryotic hosts include the phoA promoter, β-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgamo (S.D.) sequence operably linked to the DNA encoding the anti-ErbB2 antibody.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-ErbB2 antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the action promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et al., Nature, 297:598-601 (1982) on expression of human β-interferon cDNA in mouse cells under the control of a thymidine kinase promoter from herpes simplex virus. Alternatively, the rous sarcoma virus long terminal repeat can be used as the promoter.

### Enhancer element component

Transcription of a DNA encoding the anti-ErbB2 antibody of this invention by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature, 297:17-18 (1982) on enhancing elements for activation of eukaryotic promoters. The enhancer may be spliced into the vector at a position 5' or 3' to the anti-ErbB2 antibody-encoding sequence, but is preferably located at a site 5' from the promoter.

### Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding anti-ErbB2 antibody. One useful transcription termination component is the bovine growth hormone polyadenylation region. See WO94/11026 and the expression vector disclosed therein.

### Selection and transformation of host cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, *e.g*., E. coli, Enterobacter, Erwinia, Klebsiella, Proteus, Salinonella, *e.g*., Salmonella typhimurium, Serratia, *e.g*., Serratia marcescans, and Shigella, as well as Bacilli such as B. subtilis and B. licheniformis (*e.g*., B. licheniformis 41P disclosed in DD 266,710 published 12 April 1989), Pseudomonas such as P. aeruginosa, and Streptomyces. One preferred E. coli cloning host is E. coli 294 (ATCC 31,446), although other strains such as E. coli B, E. coli X1776 (ATCC 31,537), and E. coli W3110 (ATCC 27,325) are suitable. These examples are illustrative rather than limiting.

In addition to prokaryotes, eukaryotic microbes such as filamentous fungi or yeast are suitable cloning or expression hosts for anti-ErbB2 antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, *e.g.,* K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderma reesia (EP 244,234); Neurospora crassa; Schwanniomyces such as Schwanniomyces occidentalis; and filamentous fungi such as, *e.g.*, Neurospora, Penicillium, Tolypocladium, and Aspergillus hosts such as A. nidulans and A. niger.

Suitable host cells for the expression of glycosylated anti-ErbB2 antibody are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, *e.g*., the L-1 variant of Autographa californica NPV and the Bm-5 strain of Bombyx mori NPV, and such viruses may be used as the virus herein according to the present invention, particularly for transfection of Spodoptera frugiperda cells.

Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Viral., 36:59 (1977)); baby hamster kidney cells (BHK, ATCC CCL 10); Chinese hamster ovary cells/-DHFR (CHO, Urlaub et al., Proc. Natl. Acad. Sci. URSA, 77:4216 (1980)); mouse sertoli cells (TM4, Mather, Biol. Reprod., 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 (1982)); MRC 5 cells; FS4 cells; and a human hepatoma line (Hep G2).

Host cells are transformed with the above-described expression or cloning vectors for anti-ErbB2 antibody production and cultured in conventional nutrient media modified as appropriate for inducing promoters, selecting transformants, or amplifying the genes encoding the desired sequences.

### Culturing the host cells

The host cells used to produce the anti-ErbB2 antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz., 58:44 (1979), Barnes et al., Anal. Biochem.,102:255 (1980), U.S. Patent Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN® drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### Purification of anti-ErbB2 antibody

When using recombinant techniques, antibodies can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology, 10:163-167 (1992) describe a procedure for isolating antibodies which are secreted to the periplasmic space of E. coli. Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth., 62:1-13 (1983)). Protein G is recommended for all mouse isotypes and for human γ3 (Guss et al., EMBO J., 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e.g*., from about 0-0.25M salt).

### Pharmaceutical Formulations

Therapeutic formulations of the antibody used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulin; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g, Zn-protein complexes); and/or non-ionic surfactants such as TWEEN™, PLURONICS™ or polyethylene glycol (PEG). Preferred lyophilized anti-ErbB2 antibody formulations are described in WO 97/04801.

The formulation herein may also contain more than one active compound as necessary for the particular indication being treated, preferably those with complementary activities that do not adversely affect each other. For example, it may be desirable to further provide antibodies which bind to EGFR, ErbB2 (*e.g.*, an antibody which binds a different epitope on ErbB2), ErbB3, ErbB4, or vascular endothelial factor (VEGF) in the one formulation. Alternatively, or additionally, the composition may further comprise a chemotherapeutic agent, cytotoxic agent, cytokine, growth inhibitory agent, anti-hormonal agent, EGFR-targeted drug, anti-angiogenic agent, and/or cardioprotectant. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

### Treatment with And-ErbB2 Antibodies

It is contemplated that anti-ErbB2 antibody the Mab2C4 is used to treat (breast cancer, lung cancer, ovarian cancer, including advanced, refractory or recurrent ovarian cancer, and prostate cancer).

The cancer will generally comprise ErbB2-expressing cells, such that the anti-ErbB2 antibody herein is able to bind to the cancer. While the cancer may be characterized by overexpression of the ErbB2 receptor, the present application further provides for treating cancer which is not considered to be an ErbB2-overexpressing cancer. To determine ErbB2 expression in the cancer, various diagnostic/prognostic assays are available. ErbB2 overexpression may be analyzed by IHC, e.g. using the HERCEPTEST^{®} (Dako). Parrafin embedded tissue sections from a tumor biopsy may be subjected to the IHC assay and accorded a ErbB2 protein staining intensity criteria as follows:
Score 0
   no staining is observed or membrane staining is observed in less than 10% of tumor cells. Score 1+
   a faint/barely perceptible membrane staining is detected in more than 10% of the tumor cells. The cells are only stained in part of their membrane.
Score 2+
   a weak to moderate complete membrane staining is observed in more than 10% of the tumor cells.
Score 3+
   a moderate to strong complete membrane staining is observed in more than 10% of the tumor cells.

Those tumors with 0 or 1+ scores for ErbB2 overexpression assessment may be characterized as not overexpressing ErbB2, whereas those tumors with 2+ or 3+ scores may be characterized as overexpressing ErbB2.

Alternatively, or additionally, FISH assays such as the INFORM™ (sold by Ventana, Arizona) or PATHVISION™ (Vysis, Illinois) may be carried out on formalin-fixed, paraffin-embedded tumor tissue to determine the extent (if any) of ErbB2 overexpression in the tumor.

In one embodiment, the cancer will be one which expresses (and may, but does not have to, overexpress) EGFR.

The cancer to be treated herein may be one characterized by excessive activation of an ErbB receptor, e.g. EGFR. Such excessive activation may be attributable to overexpression or increased production of the ErbB receptor or an ErbB ligand.

A. diagnostic or prognostic assay may be performed to determine whether the patient's cancer is characterized by excessive activation of an ErbB receptor. For example, ErbB gene amplification and/or overexpression of an ErbB receptor in the cancer may be determined. Various assays for determining such amplification/overexpression are available in the art and include the IHC, FISH and shed antigen assays described above. Alternatively, or additionally, levels of an ErbB ligand, such as TGF-α, in or associated with the tumor may be determined according to known procedures. Such assays may detect protein and/or nucleic acid encoding it in the sample to be tested. ErbB ligand levels in the tumor may be determined using immunohistochemistry (IHC); see, for example, Scher et al., Clin. Cancer Research, 1:545-550 (1995). Alternatively, or additionally, one may evaluate levels of ErbB ligand-encoding nucleic acid in the sample to be tested; *e.g*., via FISH, southern blotting, or PCR techniques.

Moreover, ErbB receptor or ErbB ligand overexpression or amplification may be evaluated using an in vivo diagnostic assay, *e.g.,* by administering a molecule (such as an antibody) which binds the molecule to be detected and is tagged with a detectable label (*e.g.,* a radioactive isotope) and externally scanning the patient for localization of the label.

Where the cancer to be treated is hormone independent cancer, expression of the hormone (*e.g.*, androgen) and/or its cognate receptor in the tumor may be assessed using any of the various assays available, *e.g*, as described above. Alternatively, or additionally, the patient may be diagnosed as having hormone independent cancer in that they no longer respond to anti-androgen therapy.

An immunoconjugate comprising the anti-ErbB2 antibody conjugated with a cytotoxic agent may be administered to the patient. Preferably, the immunoconjugate and/or ErbB2 protein to which it is bound is/are internalized by the cell, resulting in increased therapeutic efficacy of the immunoconjugate in killing the cancer cell to which it binds. The cytotoxic agent targets or interferes with nucleic acid in the cancer cell. Examples of such cytotoxic agents include maytansinoids, calicheamicins, ribonucleases and DNA endonucleases.

The antibody for administration is rhuMAb 2C4 . RhuMAb 2C4 is a humanized monoclonal antibody based on human IgG1 framework sequences and consisting of two heavy chains (449 residues) and two light chains (214 residues). RhuMAb 2C4 differs significantly from another anti-HER2 antibody HERCEPTIN® (Trastuzumab) in the epitope-binding regions of the light chain and heavy chain. As a result, rhuMAb 2C4 binds to a completely different epitope on HER2. The present invention provides sensitive methods for identifying cancers responsive to treatment with rhuMAb 2C4 or functional equivalents thereof. It is noted that such cancers responsive to rhuMAb 2C4 treatment are not required to overexpress HER2.

The anti-ErbB2 antibodies or immunoconjugates are for administration to a human patient in accord with known methods, such as intravenous administration, e.g., as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred.

Other therapeutic regimens may be combined with the administration of the anti-ErbB2 antibody. The combined administration includes coadministration, using separate formulations or a single pharmaceutical formulation, and consecutive administration in either order, wherein preferably there is a time period while both (or all) active agents simultaneously exert their biological activities.

The patient may be treated with two different anti-ErbB2 antibodies. For example, the patient may be treated with rhu hAb 2C4 as a first anti-ErbB2 antibody as well as a second anti-ErbB2 antibody which is growth inhibitory (e.g. HERCEPTIN^{®}) or an anti-ErbB2 antibody which induces apoptosis of an ErbB2-overexpressing cell (*e.g*., 7C2, 7F3 or humanized variants thereof). Preferably such combined therapy results in a synergistic therapeutic effect. One may, for instance, treat the patient with HERCEPTIN^{®} and thereafter treat with rhuMAb 2C4, *e.g.,* where the patient does not respond to HERCEPTIN^{®} therapy. The patient may first be treated with rhuMAb 2C4 and then receive HERCEPTIN^{®} therapy. The patient may be treated with both rhuMAb 2C4 and HERCEPTIN^{®} simultaneously.

It may also be desirable to combine administration of the anti-ErbB2 antibody with administration of an antibody directed against another tumor associated antigen. The other antibody in this case may, for example, bind to EGFR, ErbB3, ErbB4, or vascular endothelial growth factor (VEGF).

The treatment may involve the combined administration of the anti-ErbB2 antibody and one or more chemotherapeutic agents or growth inhibitory agents, including coadministration of cocktails of different chemotherapeutic agents. Preferred chemotherapeutic agents include taxanes (such as paclitaxel and docetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992).

The antibody may be combined with an anti-hormonal compound; *e.g.*, an anti-estrogen compound such as tamoxifen; an anti-progesterone such as onapristone (see, EP 616 812); or an anti-androgen such as flutamide, in dosages known for such molecules. Where the cancer to be treated is hormone independent cancer, the patient may previously have been subjected to anti-hormonal therapy and, after the cancer becomes hormone independent, the anti-ErbB2 antibody (and optionally other agents as described herein) may be administered to the patient.

Sometimes, it may be beneficial to also coadminister a cardioprotectant (to prevent or reduce myocardial dysfunction associated with the therapy) or one or more cytokines to the patient. One may also coadminister an EGFR- targeted drug or an anti-angiogenic agent. In addition to the above therapeutic regimes, the patient may be subjected to surgical removal of cancer cells and/or radiation therapy.

The anti-ErbB2 antibodies herein may also be combined with an EGFR-targeted drug such as those discussed above in the definitions section resulting in a complementary, and potentially synergistic, therapeutic effect.

Examples of additional drugs which can be combined with the antibody include chemotherapeutic agents such as carboplatin, a taxane (*e.g.*, paclitaxel or docetaxel), gemcitabine, navelbine, cisplatin, oxaliplatin, or combinations of any of these such as carboplatin/docetaxel; another anti-HER2 antibody (*e.g.*, a growth inhibitory anti-HER2 antibody such as HERCEPTIN®, or an anti-HER2 antibody which induces apoptosis such as 7C2 or 7F3, including humanized or affinity matured variants thereof); a farnesyl transferase inhibitor; an anti-angiogenic agent (*e.g.*, an anti-VEGF antibody); an EGFR-targeted drug (*e.g.*, C225 or ZD1839); a cytokine (*e.g.*, IL-2, IL-12, G-CSF or GM-CSF); or combinations of the above.

Suitable dosages for any of the above coadministered agents are those presently used and may be lowered due to the combined action (synergy) of the agent and the anti-ErbB2 antibody, rhuMAb 2C4.

For the prevention or treatment of disease, the appropriate dosage of antibody will depend on the type of disease to be treated, as defined above, the severity and course of the disease, whether the antibody is administered for preventive or therapeutic purposes, previous therapy, the patient's clinical history and response to the antibody, and the discretion of the attending physician.

The doses are for administration intermittently every three weeks (*e.g.*, such that the patient receives from about two to about twenty, *e.g.*, about six doses of the anti-ErbB2 antibody). An initial higher loading dose, followed by one or more lower doses may be administered.

The progress of this therapy is easily monitored by conventional techniques and assays.

The rhuMAb 2C4 is for administration in a fixed dose of 420 mg (equivalent to doses of 6 mg/kg for a 70-kg subject) every 3 weeks. Treatment may start with a higher loading dose (*e.g.*, 840 mg, equivalent to 12 mg/kg of body weight) in order to achieve steady state serum concentrations more rapidly. Specific dosing regimens are also provided in the Examples below.

### Articles of Manufacture

Also disclosed is an article of manufacture containing materials useful for the treatment of the disorders described above is provided.

The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is an anti-ErbB2 antibody. The label or package insert indicates that the composition is used for treating the condition of choice, such as cancer. The label or package inserts may indicate that the composition comprising the antibody which binds ErbB2 can be used to treat a patient suffering from a tumor in which the presence of HER2/HER1 and/or HER2/HER3 and/or HER2/HER4 complexes have been identified, and/or phosphorylation of ErbB receptor has been detected. Moreover, the article of manufacture may comprise (a) a first container with a composition contained therein, wherein the composition comprises a first antibody which binds ErbB2 and inhibits growth of cancer cells which overexpress ErbB2; and (b) a second container with a composition contained therein, wherein the composition comprises a second antibody which binds ErbB2 and blocks ligand activation of an ErbB receptor. The article of manufacture may further comprises a package insert indicating that the first and second antibody compositions can be used to treat cancer characterized by the presence of HER2/HER1 and/or HER2/HER3 and/or HER2/HER4 heterodimers, and/or by the phosphorylation of ErbB receptor. Moreover, the package insert may instruct the user of the composition (comprising an antibody which binds ErbB2 and blocks ligand activation of an ErbB receptor) to combine therapy with the antibody and any of the adjunct therapies described in the preceding section (*e.g.* a chemotherapeutic agent, an EGFR-targeted drug, an anti-angiogenic agent, an anti-hormonal compound, a cardioprotectant and/or a cytokine). Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Deposit of Materials

The following hybridoma cell lines have been deposited with the American Type Culture Collection, 10801 University Boulevard, Manassas, VA 20110-2209, USA (ATCC):

| **Antibody Designation** | **ATCC No.** | **Deposit Date** |
|---|---|---|
| 7C2 | ATCC HB-12215 | October 17, 1996 |
| 7F3 | ATCC HB-12216 | October 17, 1996 |
| 4D5 | ATCC CRL 10463 | May 24, 1990 |
| 2C4 | ATCC HB-12697 | April 8, 1999 |

Further details of the invention are illustrated by the following non-limiting Examples.

### Example 1

### HRG Dependent Association of ErbB2 with ErbB3 is Blocked by Monoclonal Antibody 2C4

The murine monoclonal antibody 2C4, which specifically binds the extracellular domain of ErbB2 is described in WO 01/89566.

The ability of ErbB3 to associate with ErbB2 was tested in a co-immunoprecipitation experiment. 1.0 x 106 MCF7 or SK-BR-3 cells were seeded in six well tissue culture plates in 50:50 DMEM/Ham's F12 medium containing 10% fetal bovine serum (FBS) and 10 mM HEPES, pH 7.2 (growth medium), and allowed to attach overnight. The cells were starved for two hours in growth medium without serum prior to beginning the experiment

The cells were washed briefly with phosphate buffered saline (PBS) and then incubated with either 100 nM of the indicated antibody diluted in 0.2% w/v bovine serum albumin (BSA), RPMI medium, with 10 mM HERPES, pH 7.2 (binding buffer), or with binding buffer alone (control). After one hour at room temperature, HRG was added to a final concentration of 5 nM to half the wells (+). A similar volume of binding buffer was added to the other wells (-). The incubation was continued for approximately 10 minutes.

Supernatants were removed by aspiration and the cells were lysed in RPMI, 10 mM HEPES, pH 7.2, 1.0% v/v TRITON X-100™, 1.0% w/v CHAPS (lysis buffer), containing 0.2 mM PMSF, 10 µg/ml leupeptin, and 10 TU/ml aprotinin. The lysates were cleared of insoluble material by centrifugation.

ErbB2 was immunoprecipitated using a monoclonal antibody covalently coupled to an affinity gel (Affi-Prep 10, Bio-Rad). This antibody (Ab-3, Oncogene Sciences, USA) recognizes a cytoplasmic domain epitope. Immunoprecipitation was performed by adding 10 µl of gel slurry containing approximately 8.5 µg of immobilized antibody to each lysate, and the samples were allowed to mix at room temperature for two hours. The gels were then collected by centrifugation. The gels were washed batchwise three times with lysis buffer to remove unbound material. SDS sample buffer was then added and the samples were heated briefly in a boiling water bath.

Supernatants were run on 4-12% polyacrylamide gels and electroblotted onto nitrocellulose membranes. The presence of ErbB3 was assessed by probing the blots with a polyclonal antibody against a cytoplasmic domain epitope thereof (c-17, Santa Cruz Biotech). The blots were visualized using a chemiluminescent substrate (ECL, Amersham)

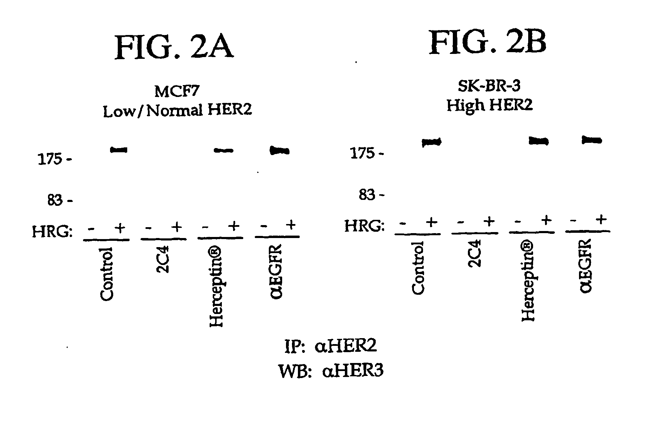

As shown in the control lanes of Figs. 2A and 2B, for MCF7 and SK-BR-3 cells, respectively, ErbB3 was present in an ErbB2 immunoprecipitate only when the cells were stimulated with HRG. If the cells were first incubated with monoclonal antibody 2C4, the ErbB3 signal was abolished in MCF7 cells (Fig. 5A, lane 2C4 +) or substantially reduced in SK-BR-3 cells (Fig. 5B, lane 2C4+). As shown in Figs 2A-B, monoclonal antibody 2C4 blocks heregulin dependent association of ErbB3 with ErbB2 in both MCF7 and SK-BR-3 cells substantially more effectively than HERCEPTIN^{®}. Preincubation with HERCEPTIN^{®} decreased the ErbB3 signal in MCF7 lysates but had little or no effect on the amount of ErbB3 co-precipitated from SK-BR-3 lysates. Preincubation with an antibody against the EGF receptor (Ab-1, Oncogene Sciences, USA) had no effect on the ability of ErbB3 to co-immunoprecipitate with ErbB2 in either cell line.

### Example 2

### Responsiveness of Cell Line and Human Tumor Xenograft Models to 2C4

Approximately 40 tumor models have been tested for responsiveness to 2C4. These models represent major cancers such as breast, lung, prostate and colon. 50-60% of the models responded to 2C4 treatment. Table 1 below lists selected tumor models tested for responsivity to 2C4. Briefly, human tumor xenograft fragments of about 3 mm size were transplanted underneath the skin of athymic nude mice. Alternatively, human tumor cells grown in vitro were detached from the culture dishes, resuspended in phosphate-buffered saline and subcutaneously injected into the flank of the immuno-compromised mice. The growth of tumors was monitored every 2 to 3 days using an electric caliper. When the tumors reached a size of about 30 to 100 mm, animals were randomized into different treatment and control groups. 2C4 was administered by intraperitoneal injection once every week. Control animals received equal volumes of vehicle solution containing no antibody on the same schedule as the treatment groups. The study was terminated after approximately 3-6 weeks, when the tumors of the control group reached a size of about 1000 - 1500 mm3. Responsiveness to treatment was defined as ≥ 50% tumor volume reduction.

**Table 1: Xenograft models**

| **Model #** | **Tumor model** | **Responsiveness to 2C4** | **Reference** |
|---|---|---|---|
| 1 | LXFA 289 | No | (Fiebig et al., 1999) |
| 2 | LXFA 297 | Yes | |
| 3 | LXFA 526 | No | (Fiebig et al., 1999) |
| 4 | LXFA 629 | Yes | (Fiebig and Burger, 2002) |
| 5 | LXFA 1041 | No | |
| 6 | LXFE 211 | No | (Fiebig et al., 1999) |
| 7 | LIFE 397 | No | (Fiebig et al., 1999) |
| 8 | LXFL 529 | No | (Burger et al., 2001) |
| 9 | LXFL 1072 | Yes | (Fiebig et al., 1999) |
| 10 | Calu-3 | Yes | (Stein et al., 2001) |
| 11 | NCI-H522 | Yes | (Yamori et al., 1997) |
| 12 | NCI-H322 | No | (Zou et al., 2001) |
| 13 | NCI-H441(KAM) | Yes | (Gridley et al., 1996) |
| 14 | MAXF MX1 | No | |
| 15 | MAXF 401 | No | (Fiebig et al., 1999) |
| 16 | MAXF 449 | Yes | (Burger et al., 2001) |
| 17 | MAXF 713 | No | (Berger et al., 1992) |
| 18 | MAXF 857 | No | (Fiebig et al., 1999) |

The models represent two major tumor types, namely non-small cell lung cancer (NSCLC; models #1-13) and mammary cancer (models #14-18). Nine of the NSCLC models (#1-9) and all breast cancer models were derived by serial in vivo passage of human tumor fragments in immunodeficient mice. The remaining NSCLC models (#10-13) are cell-based models in which in vivo tumor growth is induced by implantation of in vitro propagated cells into immunocompromised mice.

Berger, D. P., Winterhalter, B. R., and Fiebig, H. H. (1992). Establishment and Characterization of Human Tumor Xenografts in Thymus-Aplastic Nude Mice. In Immunodeficient Mice in Oncology, H. H. Fiebig and D. P. Berger, eds. (Basel: Karger), pp. 23-46.

Burger, A. M., Hartung, G., Stehle, G., Sinn, H., and Fiebig, H. H. (2001). Pre-clinical evaluation of a methotrexate-albumin conjugate (MTX-HSA) in human tumor xenografts in vivo. Int. J. Cancer, 92:718-24.

Fiebig, H. H., and Burger, A. M. (2002). Human Tumor Xenografts and Explants. In Tumor Models in Cancer Research, B. A. Teicher, ed. (Totowa, New Jersey: Humana Press), pp. 113-137.

Fiebig, H. H., Dengler, W. A., and Roth, T. (1999). Human Tumor Xenografts: Predictivity, Characterization and Discovery of New Anticancer Agents. In Contributions to Oncology: Relevance of Tumor Models for Anticancer Drug Development, H. H. Fiebig and A. M. Burger, eds. (Basel: Karger), pp. 29-50.

Gridley, D. S., Andres, M. L., Garner, C., Mao, X. W., and Slater, J. M. (1996). Evaluation of TNF-alpha effects on radiation efficacy in a human lung adenocarcinoma model. Oncol Res., 8:485-95.

Stein, R., Govindan, S. V., Chen, S., Reed, L., Spiegelman, H., Griffiths, G. L., Hansen, H. J., and Goldenberg, D. M. (2001). Successful therapy of a human lung cancer xenograft using MAb RS7 labeled with residualizing radioiodine. Crit. Rev. Oncol. Hematol., 39:173-80:

Yamori, T., Sato, S., Chikazawa, H., and Kadota, T. (1997). Anti-tumor efficacy of paclitaxel against human lung cancer xenografts. Jpn. J. Cancer Res., 88:1205-10.

Zou, Y., Wu, Q. P., Tansey, W., Chow, D., Hung, M. C., Charnsangavej, C., Wallace, S., and Li, C. (2001). Effectiveness of water soluble poly(L-glutamic acid)-camptothecin conjugate against resistant human lung cancer xenografted in nude mice. Int. J. Oncol., 18:331-6.

### Example 3

### Detection of Heterodimers in 2C4 Responsive Tumors by Immunoprecipitation

2C4 responsive and non-responsive tumors were subjected to immunoprecipitation with anti-ErbB2 antibodies to assay for the presence of ErbB2-ErbB3 and EGFR-ErbB2 heterodimers. Unless otherwise stated the methods were performed according to Maniatis T. et al, Molecular Cloning: A Laboratory Manual, Cold Spring Harbor, New York, USA, Cold Spring Harbor Laboratory Press, 1982.

Anti-HER2, anti-HER3 and anti-HER1 antibodies were selected that did not cross react. To determine if antibodies cross reacted, HER1, HER2, HER3 and HER4 receptors were expressed in human embryonic kidney (HEK) 293 cells. The cells were lysed using a Triton™ X100 (1% weight per volume) containing HEPES buffer (pH 7.5). Approximately 20 µg of total cell protein from control cells and HER1, HER2, HER3 and HER4 expressing cells was separated on an SDS gel and transferred to a nitrocellulose membrane by a semi-dry blotting procedure. After blocking with gelatin, a variety of anti-HER1, anti-HER2 and anti-HER3 antibodies were tested for their cross reactivity against other ErbB receptors. Antibodies selected for use in the experiments described below did not show any significant cross reactivity.

Fresh tumor samples were crushed mechanically on ice and lysed in a buffer containing 50 mM HEPES pH 7.5, 150 mM NaCl, 1.5 mM MgCl₂, 1 mM EDTA, 10 % (w/v) glycerol, 1 % (w/v) Triton™ X-100, 1 mM PMSF, 10 µg/ml aprotinin and 0.4 mM orthovanadate. Completely lysed tumors were centrifuged several times until the supernatants were completely clear. Immunoprecipitation proceeded by combining cleared tumor lysates (5-7 mg protein per lysate), 5 µg anti ErbB2 antibody (ab-3, mouse monoclonal; Cat.# OP15, Oncogene Inc., USA) and 50 µl protein G-coupled agarose in 1.5 ml Eppendorf reaction tubes. Upon addition of a one to twofold volume of 50 mM HEPES buffer, pH 7.5 containing 0.1 % (w/v) Triton™ X-100 the tubes were rotated for 3-4 hours at 4 °C followed by centrifugation. The pellets were washed two to three times with 500 µl of 50 mM HEPES buffer pH 7.5 containing 0.1 % (w/v) Triton™ X-100. An equal volume of 2x Lämmli sample buffer was added to the washed immunoprecipitate and the samples were heated for 5 min at 95°C. The samples were separated by SDS-PAGE and transferred to nitrocellulose. The presence of EGFR-ErbB2 and ErbB2-ErbB3 heterodimers was assessed by probing the blots with antibodies against EGFR (rabbit polyclonal antibody against EGFR, Upstate Inc., USA; Cat. # 06-847) and ErbB3 ( polyclonal antibody against ErbB3; Santa Cruz Inc., USA; Cat. # SC-285).). An peroxidase (POD) labeled anti-rabbit Fc antibody (BioRad Laboratories Inc. USA) was used as secondary antibody. The blots were visualized using a chemiluminescence substrate (ECL plus, Amersham).

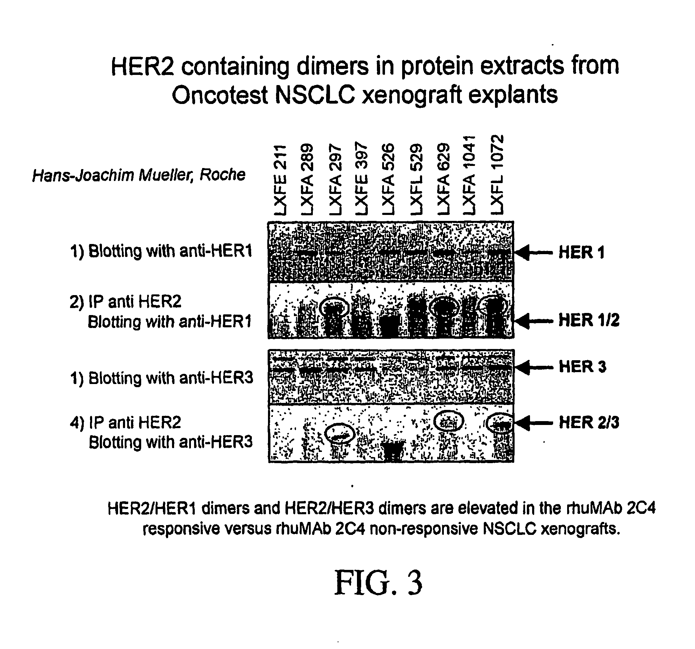

Figure 3 shows the results of these experiments. The presence of ErbB2-ErbB3 and/or EGFR-ErbB2 heterodimers can be seen. A correlation was observed between 2C4 responsiveness, as shown in Table 1, and the presence of ErbB2-ErbB3 and/or EGFR-ErbB2 heterodimers.

### Example 4

### Correlation of Responsiveness to rhuMAb 2C4 with HER2 Phosphorylation

The effect of rhuMAb 2C4 on tumor growth has been studied in 14 human tumors explanted into mice (9 lung cancer and 5 breast cancer). The explantation of tumor and treatment were performed as described in Example 2. HER2 heterodimers were detected as described in Example 3.

HER2 phosphorylation was assessed by immunoprecipitation of HER2 and Western blot analysis. Positivity was determined by the presence of the phospho-HER2 band of the gel. Negativity was determined by the absence of the band. HER2 phosphorylation was confirmed by immunohistochemistry using a phospho-specific anti-HER2 antibody (clone PN2A, Thor et al., J. Clin. Oncol., 18:3230-9 (2000).

In 5 of the tumors tested (3 lung and 2 breast), a significant inhibition of tumor growth was observed, which correlated with the presence of detectable heterodimers of HER2 with either HER1 or HER3, and with strong HER2 phosphorylation in all cases. In 9 tumors in which no significant response to rhuMAb 2C4 treatment was observed, heterodimers were not detected and HER2 phosphorylation was absent. The presence of HER2 heterodimerization or significant HER2 phosphorylation is a strong predictor of response to rhuMAb 2C4 treatment in nonclinical models. Similar observations have been made with xenografts generated from tumor cell lines.

### Example 5

### Detection of HER2 Phosphorylation in 2C4 Responsive Tumors

The efficacy of rhuMAb 2C4 was assessed in nine established non-small cell lung carcinoma (NSCLC) xenografted tumor models (LXFE 211, LXFA 289, LXFA 297, LXFE 397, LXFA 526, LXFL 529, LXFA 629, LXFA 1041, LXFL 1071, Oncotest GmbH, Freiburg, Germany). Human tumor xenografts are being considered as the most relevant models for anticancer drug development since the patient's tumors are growing as a solid tumor, develop a stroma, vasculature, a central necrosis and show dome differentiation. In addition, the xenografted tumor models resemble very closely the original tumors in histology and chemosensitivity.

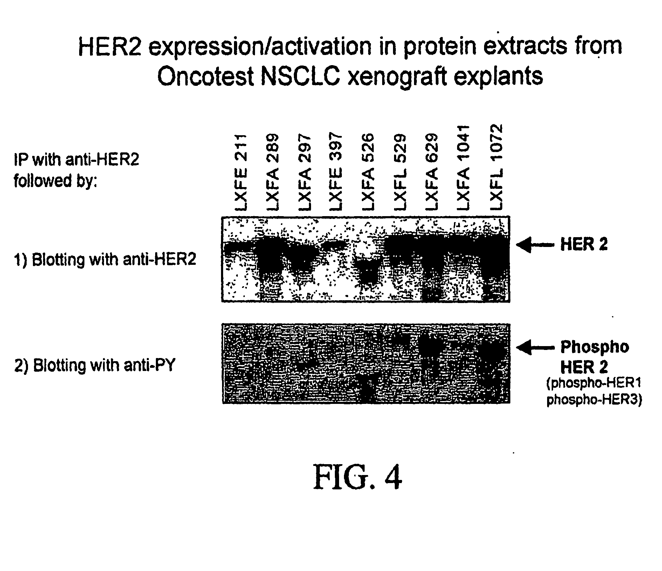

Growth inhibition was assessed as described in Example 2. Significant growth inhibitory activity was defined as >50% growth inhibition of the treatment group relative to the control group. In three of the NSCLC models (LXFA 297, LXFA 629, and LXFL 1072), a significant growth inhibitory response to rhuMAb 2C4 treatment was observed. Several hallmarks of ligand-activated HER2 have also been investigated at the protein level. As shown in Figure 4, HER2 could be immunoprecipitated from tumor extracts from eight of nine NSCLC tumors. To determine the activation status of HER2, these blots were then probed with anti-phosphotyrosine (anti-PY) antibody. As shown in the lower panel of Figure 4, the three responsive tumors (LXFA 297, LXFA 629 and 1072) displayed strong HER2 activation.

### Example 6

### Clinical Study to Identify Lung Cancer Patients for Treatment with rhuMAb 2C4 by Detecting HER2 Heterodimers

A patient is identified as having non-small cell lung cancer (NSCLC) that is responsive to treatment with rhuMAb 2C4 if a tumor from the patient is found to comprise HER2/HER3 and/or HER2/HER1 and/or HER2/HER4 complexes.

A tumor sample is obtained by biopsy or during surgical resection of the tumor. The sample is then analyzed for the presence of HER2/HER3 and/or HER2/HER1 and/or HER2/HER4 heterodimers.

Tumor cells or cell lysates are contacted with an eTag™ that specifically binds HER2. The eTag™ comprises a detectable moiety and a first binding moiety that is specific for HER2. The detectable moiety is linked to the first binding moiety with a cleavable linker. After allowing time for binding, excess eTag™ is removed.

The tumor cells or cell lysates are contacted with a second binding compound that specifically binds HER1 or HER3 or HER4. Unbound compound is removed by washing. The second binding compound is then activated. If the first binding compound and the second binding compound are in close proximity, the activated second binding compound cleaves the cleavable linker in the eTag™ to produce a free detectable moiety. The identification of free detectable moiety in the sample indicates that the tumor comprises HER2/HER1 or HER2/HER3 or HER2/HER4 heterodimers.

Upon determination that the patient is suffering from a tumor that comprises HER2/HER1 and/or HER2/HER3 and/or HER2/HER4 heterodimers, rhuMAb 2C4 is administered intravenously (IV) weekly or every three weeks at 2 or 4 mg/kg, respectively, until disease progression. The antibody is supplied as a multi-dose liquid formulation (20mL fill at a concentration of 20mg/mL or higher concentration). Primary endpoints for efficacy include response rate, and safety. Secondary efficacy endpoints include: overall survival, time to disease progression, quality of life, and/or duration of response.

### Example 7

### Clinical Study to Identify Cancer Patients for Treatment with rhuMAb 2C4

A biological sample comprising cancer cells is obtained from candidates for the treatment, *e.g.*, by biopsy of tumor tissue, aspiration of tumor cells from ascitic fluid, or any other method known in clinical practice. The biological sample is analyzed for HER2 phosphorylation, *e.g.*, by immunoprecipitation and Western blot analysis, and/or for the presence of HER2/HER3, HER2/HER1 and/or HER2/HER4 heterodimers by any of the techniques described above. Subjects whose biological sample was positive for HH2 phosphorylation and/or the presence of HER2/HER3, HER2/HER1 and/or HER2/HER4 heterodimers, are likely to show a better response to treatment with rhuMAb 2C4 than patients whose tumor sample showed no HER2 phosphorylation or where no heterodimer was detected.

For example, subjects diagnosed with ovarian cancer will undergo a biopsy of tumor tissue or aspiration of tumor cells from ascites fluid. This tissue will be analyzed for HER2 phosphorylation by immunoprecipitation and Western blot analysis. This will require a minimum of about 250 mg tumor tissue.

Upon determination that the patient suffers from cancer (such as, Castration-Resistant Prostate Cancer - CRPC, or ovarian cancer) that is positive for HER2 phosphorylation, the patient will receive a loading dose of 840 mg of rhuMAb 2C4 on day 1 of cycle 1 (first 21-day treatment period), followed by 420 mg on day 1 of each subsequent 21-day cycle, as continuous intravenous infusion. Treatment continues by intravenous infusion every 3 weeks for up to one year (17 cycles), for patients who show no evidence of disease progression. Treatment can be discontinued any time earlier, for lack of response, adverse effects, or other reasons, at the discretion of the physician.

## Claims

1. Recombinant humanized antibody 2C4 (rhuMAb 2C4) for use in a method for treatment of ovarian, breast, lung, or prostate cancer in a cancer patient, wherein the rhuMAb 2C4 comprises the variable light and variable heavy amino acid sequences of SEQ ID NOs. 3 and 4, respectively, and human light and heavy IgG1 (non-A allotype) constant region sequences, and wherein in the method rhuMAb 2C4 is administered as a fixed dose of 420mg every three weeks.

2. The recombinant humanized antibody for use according to claim 1 wherein the rhuMAb 2C4 is Chinese Hamster Ovary (CHO) cell expressed.

3. The recombinant humanized antibody for use according to claim 1 or claim 2, wherein the treatment with rhuMAb 2C4 starts with a 840mg loading dose.

4. Use of a recombinant humanized antibody 2C4 (rhuMAb 2C4) in the preparation of a medicament for the treatment of ovarian, breast, lung, or prostate cancer in a cancer patient, wherein the rhuMAb 2C4 comprises the variable light and variable heavy amino acid sequences of SEQ ID NOs. 3 and 4, respectively, and human light and heavy IgG1 (non-A allotype) constant region sequences, and wherein in the treatment rhuMAb 2C4 is administered as a fixed dose of 420mg every three weeks.

5. Use according to claim 4 wherein the rhuMAb 2C4 is Chinese Hamster Ovary (CHO) cell expressed.

6. Use according to claim 5 wherein the medicament is for treatment of a cancer patient wherein the treatment with rhuMAb 2C4 starts with a 840mg loading dose.

## Patentansprüche

1. Rekombinanter humanisierter Antikörper 2C4 (rhuMAb 2C4) zur Verwendung in einem Verfahren zur Behandlung von Ovarial-, Brust-, Lungen- oder Prostatakrebs bei einem Krebspatienten, wobei der rhuMAb 2C4 die variable leichte und variable schwere Aminosäuresequenz der Seq.-ID Nr. 3 bzw. 4 und die menschliche leichte und schwere Sequenz der konstanten IgG1-Region (Nicht-A-Allotyp) umfasst und wobei rhuMAb 2C4 im Verfahren alle drei Wochen als fixe Dosis von 420 mg verabreicht wird.

2. Rekombinanter humanisierter Antikörper zur Verwendung nach Anspruch 1, wobei der rhuMAb 2C4 von einer Chinahamster-Ovarialzelle (CHO-Zelle) exprimiert wird.

3. Rekombinanter humanisierter Antikörper zur Verwendung nach Anspruch 1 oder Anspruch 2, wobei die Behandlung mit rhuMAb 2C4 mit einer Belastungsdosis von 840 mg beginnt.

4. Verwendung eines rekombinanten humanisierten Antikörpers 2C4 (rhuMAb 2C4) zur Herstellung eines Medikaments zur Behandlung von Ovarial-, Brust-, Lungen- oder Prostatakrebs bei einem Krebspatienten, wobei der rhuMAb 2C4 die variable leichte und variable schwere Aminosäuresequenz der Seq.-ID Nr. 3 bzw. 4 und die menschliche leichte und schwere Sequenz der konstanten IgG1-Region (Nicht-A-Allotyp) umfasst und wobei rhuMAb 2C4 bei der Behandlung alle drei Wochen als fixe Dosis von 420 mg verabreicht wird.

5. Verwendung nach Anspruch 4, wobei der rhuMAb 2C4 von einer Chinahamster-Ovarialzelle (CHO-Zelle) exprimiert wird.

6. Verwendung nach Anspruch 5, wobei das Medikament zur Behandlung eines Krebspatienten dient, wobei die Behandlung mit rhuMAb 2C4 mit einer Belastungsdosis von 840 mg beginnt.

## Revendications

1. Anticorps humanisé recombinant 2C4 (rhuMAb 2C4) pour utilisation dans une méthode de traitement du cancer des ovaires, du sein, du poumon ou de la prostate chez un patient atteint d'un cancer, ledit rhuMAb 2C4 comprenant les séquences d'amino-acides de chaînes légères variables et chaînes lourdes variables des SEQ ID Nos 3 et 4, respectivement, et les séquences de régions constantes de chaînes légères et lourdes humaines IgG1 (allotype non-A), méthode dans laquelle le rhuMAb 2C4 est administré à une dose fixe de 420 mg toutes les trois semaines.

2. Anticorps humanisé recombinant pour son utilisation suivant la revendication 1, ledit rhuMAb 2C4 étant exprimé dans des cellules d'ovaires de hamster chinois (CHO).

3. Anticorps humanisé recombinant pour son utilisation suivant la revendication 1 ou la revendication 2, le traitement avec le rhuMAb 2C4 débutant avec une dose d'attaque de 840 mg.

4. Utilisation d'un anticorps humanisé recombinant 2C4 (rhuMAb 2C4) dans la préparation d'un médicament pour le traitement du cancer des ovaires, du sein, du poumon ou de la prostate chez un patient atteint d'un cancer, dans laquelle le rhuMAb 2C4 comprend les séquences d'aminoacides de chaînes légères variables, les chaînes lourdes variables des SEQ ID Nos 3 et 4, respectivement, et les séquences de régions constantes de chaînes légères et lourdes humaines IgG1 (allotype non-A), et dans laquelle, dans le traitement, rhuMAb 2C4 est administré à une dose fixe de 420 mg toutes les trois semaines.

5. Utilisation suivant la revendication 4, dans laquelle le rhuMAb 2C4 est exprimé dans des cellules d'ovaires de hamster chinois (CHO).

6. Utilisation suivant la revendication 5, dans laquelle le médicament est destiné au traitement d'un patient atteint d'un cancer, dans laquelle le traitement avec le rhuMAb 2C4 débute avec une dose d'attaque de 840 mg.


### SEQUENCE LISTING

<110> Genentech, Inc.
   Koll, Hans
   Bossenmaier, Birgit
   Muller, Hans-Joachim
   Sliwkowski, Mark
   Kelsey, Stephen
<120> Methods For Identifying Tumors That Are
   Responsive To Treatment with Anti-ErbB2 Antibodies
<130> 39766-0114PC
<150> US 60/396,290
   <151> 2002-07-15
<150> US 60/480,043
   <151> 2003-06-20
<160> 6
<170> FastSEQ for windows version 4.0
<210> 1
   <211> 107
   <212> PRT
   <213> Homosapiens
<400> 1
   Asp Thr Val Met Thr Gln Ser His Lys Ile Met Ser Thr Ser Val Gly
    1               5                  10                  15
   Asp Arg Val Ser Ile Thr Cys Lys Ala Ser Gln Asp Val Ser Ile Gly
               20                  25                  30
   Val Ala Trp Tyr Gln Gln Arg Pro Gly Gln Ser Pro Lys Leu Leu Ile
           35                  40                  45
   Tyr Ser Ala Ser Tyr Arg Tyr Thr Gly Val Pro Asp Arg Phe Thr Gly
       50                  55                  60
   Ser Gly Ser Gly Thr Asp Phe Thr Phe Thr Ile Ser Ser Val Gln Ala
   65                  70                  75                  80
   Glu Asp Leu Ala Val Tyr Tyr Cys Gln Gln Tyr Tyr Ile Tyr Pro Tyr
                   85                  90                  95
   Thr Phe Gly Gly Gly Thr Lys Leu Glu Ile Lys
               100                 105
<210> 2
   <211> 119
   <212> PRT
   <213> Homosapiens
<400> 2
   Glu Val Gln Leu Gln Gln Ser Gly Pro Glu Leu Val Lys Pro Gly Thr
    1               5                  10                  15
   Ser Val Lys Ile Ser Cys Lys Ala Ser Gly Phe Thr Phe Thr Asp Tyr
               20                  25                  30
   Thr Met Asp Trp Val Lys Gln Ser His Gly Lys Ser Leu Glu Trp Ile
           35                  40                  45
   Gly Asp Val Asn Pro Asn Ser Gly Gly Ser Ile Tyr Asn Gln Arg Phe
       50                  55                  60
   Lys Gly Lys Ala Ser Leu Thr Val Asp Arg Ser Ser Arg Ile Val Tyr
   65                  70                  75                  80
   Met Glu Leu Arg Ser Leu Thr Phe Glu Asp Thr Ala Val Tyr Tyr Cys
                   85                  90                  95
   Ala Arg Asn Leu Gly Pro Ser Phe Tyr Phe Asp Tyr Trp Gly Gln Gly
               100                 105                 110
   Thr Thr Leu Thr Val Ser Ser
           115
<210> 3
   <211> 107
   <212> PRT
   <213> Homosapiens
<400> 3
<210> 4
   <211> 119
   <212> PRT
   <213> Homosapiens
<400> 4
<210> 5
   <211> 107
   <212> PRT
   <213> Homosapiens
<400> 5
<210> 6
   <211> 119
   <212> PRT
   <213> Homosapiens
<400> 6
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ala Val Ile Ser Gly Asp Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Arg Val Gly Tyr Ser Leu Tyr Asp Tyr Trp Gly Gln Gly
               100                 105                 110
   Thr Leu Val Thr Val Ser Ser
           115